# EUROPÄISCHE PATENTSCHRIFT

(11) **EP 4 412 545 B1**
(45) Veröffentlichungstag und Bekanntmachung des Hinweises auf die Patenterteilung: **25.02.2026**
(21) Anmeldenummer: 22808640.1
(22) Anmeldetag: 18.10.2022
(51) Int. Cl.: A61B 17/72

(54) **GEKRÜMMTER MARKNAGEL**
CURVED INTRAMEDULLARY NAIL
CLOU MÉDULLAIRE INCURVÉ

(30) Priorität: 19.10.2021 DE 102021127020
(43) Veröffentlichungstag der Anmeldung: 14.08.2024
(73) Patentinhaber: Betz, Augustin, 66687 Wadern-Wadrill (DE)
(72) Erfinder: Betz, Augustin, 66687 Wadern-Wadrill (DE)
(74) Vertreter: Manitz Finsterwald Patent- und Rechtsanwaltspartnerschaft mbB
(86) Internationale Anmeldenummer: PCT/EP2022/078929
(87) Internationale Veröffentlichungsnummer: WO 2023/066909

(56) Entgegenhaltungen:
- WO-A1-2020/187463
- WO-A2-2020/152171

## Beschreibung

Die Erfindung betrifft einen Marknagel, insbesondere einen Distraktionsmarknagel oder einen Kontraktionsmarknagel, zum Verbinden zweier Teile eines Knochens, welcher insbesondere zum sukzessiven Verlängern oder zum sukzessiven Verkürzen eines Knochens vorgesehen sein kann.

Grundsätzlich kann ein Marknagel operativ eingesetzt werden, um eine Knochenlücke zwischen zwei Knochenteilen eines durchtrennten Knochens zu überbrücken, so dass sich die Knochenteile durch eine kontinuierliche Neubildung von Knochensubstanz in der Knochenlücke wieder miteinander verbinden können. Beispielsweise können Marknägel zur Defektüberbrückung nach Trümmerbrüchen, einer Knochenentzündung oder nach einem Entfernen von Tumoren in Bereichen von langen Röhrenknochen eingesetzt werden.

Ferner können Marknägel als Distraktionsmarknägel ausgebildet sein, um zwei Knochenteile, welche mittels des Marknagels miteinander verbunden sind, durch sukzessives Herausbewegen der Stange aus dem Rohr zunehmend voneinander zu entfernen und den Knochen durch die sich während der Behandlung kontinuierlich bildende Knochensubstanz zu verlängern. Ebenso können Marknägel als Kontraktionsmarknägel ausgebildet sein, welche ein sukzessives Verkürzen des Abstandes zwischen den mit dem Marknagel verbundenen Knochenteilen ermöglichen.

Distraktionsmarknägel sind beispielsweise aus den Druckschriften DE 19 700 225 A1, DE 3 921 972 A1, US 9 179 938 B2 und CA 2 917 676 A1 bekannt. Um die gewünschte Verlängerung des Knochens zu erreichen, weisen bekannte Distraktionsmarknägel eine innerhalb des Marknagels angeordnete Einstellmechanik auf, welche von einem Arzt oder dem Patienten selbst bedient werden kann. Aufgrund dieser Anordnung der Einstellmechanik im Inneren des Marknagels ist somit keine Verbindung des Distraktionsmarknagels durch die Haut nach außen zu einer externen Einstelleinrichtung erforderlich, wodurch derartige Distraktionsmarknägel erhebliche Vorteile beispielsweise im Hinblick auf eine mögliche Infektionsgefahr im Bereich des zu verlängernden Knochens mit sich bringen.

Ein Distraktionsmarknagel mit einer Einstellmechanik, die sich als zuverlässig und belastbar erwiesen hat, ist in US 5 074 882 beschrieben. Die dort gezeigte Einstellmechanik kann betätigt werden, indem die mit dem Distraktionsmarknagel verbundenen Knochenteile um einen definierten Winkel wiederholt relativ zueinander gedreht und zurückgedreht werden, wobei die Drehung auf Komponenten der Einstellmechanik übertragen wird. Dieser mechanische Aufbau ermöglicht eine robuste Ausbildung des Distraktionsmarknagels, insbesondere gegenüber axialen Kräften.

Marknägel mit einer solchen Einstellmechanik können jedoch bislang lediglich als über ihre gesamte Länge geradlinig ausgebildet werden, ohne eine Anpassung an eine physiologische Krümmung eines Knochens zu ermöglichen. Geradlinige Marknägel können jedoch aufgrund dieses Unterschieds zu der Krümmung des Knochens operativ aufwendig einzusetzen sein und es kann im Zuge einer Operation gegebenenfalls zu einer Schwächung der Kortikalis kommen. Daher besteht ein Bedarf an Marknägeln, insbesondere Distraktionsmarknägeln, die eine Anpassung an die physiologische Krümmung eines zu behandelnden Knochens ermöglichen und dementsprechend selbst gekrümmt ausgebildet werden können.

Eine erste Realisierung eines gekrümmten Distraktionsmarknagels ist in WO 2020/152171 A1 beschrieben. Der darin beschriebene Marknagel weist ein Rohr und eine darin angeordnete, jedoch aus dem Rohr herausragende Stange auf, so dass ein erster Knochenteil an einem Ende des Rohrs und ein zweiter Knochenteil an dem entgegengesetzten Ende der Stange befestigt werden kann. Durch wiederholte Relativdrehungen zwischen der Stange und dem Rohr kann eine Einstellmechanik des Marknagels betätigt und die Stange aus dem Rohr herausbewegt werden, um den Abstand der Knochenteile zueinander sukzessive zu vergrößern. Die gewünschte Krümmung des Distraktionsmarknagels wird dadurch ermöglicht, dass - anders als bei dem aus US 5 074 882 bekannten geradlinig ausgebildeten Distraktionsmarknagel - eine axiale Kopplung zwischen der Stange und der innerhalb des Rohres angeordneten Einstellmechanik aufgegeben wird. Dadurch kann die Stange gegenüber dem Rohr ausgelenkt werden, ohne dass krümmungsbedingte Biegemomente auf die Einstellmechanik übertragen werden, die zu einer Beschädigung und gegebenenfalls sogar einem Brechen von Komponenten der Einstellmechanik führen könnten. Der operative Einsatz eines derart ausgebildeten Distraktionsmarknagels geht jedoch mit der Problematik einher, dass sich die Stange aufgrund dieser Entkopplung möglicherweise ungewollt aus dem Rohr lösen kann, wenn der Marknagel beispielsweise zur nachträglichen Aufweitung eines Markkanals nochmals entnommen werden muss.

Es ist daher eine Aufgabe der Erfindung, einen zuverlässigen, stabilen und einfach zu betätigenden Marknagel zu schaffen, welcher eine Anpassung an eine physiologische Krümmung eines Knochens sowie eine sichere Kopplung zwischen der Stange und dem Rohr ermöglicht.

Diese Aufgabe wird gelöst durch einen Marknagel mit den Merkmalen des Anspruchs 1 und insbesondere dadurch, dass der Marknagel gekrümmt ausgebildet ist und ein gekrümmtes Rohr zum Befestigen an einem ersten Knochenteil sowie eine innerhalb des Rohres angeordnete gekrümmte Stange aufweist, welche einen aus dem Rohr herausragenden Befestigungsabschnitt zum Befestigen an einem zweiten Knochenteil aufweist. Ferner ist innerhalb des Rohres eine Einstellmechanik angeordnet, welche durch wiederholte relative Drehbewegungen zwischen dem Rohr und der Stange betätigbar ist, wobei die Stange durch Betätigen der Einstellmechanik aus dem Rohr herausfahrbar oder in das Rohr hineinfahrbar ist. Die Einstellmechanik umfasst ein in einem Innengewinde des Rohres um eine Drehachse drehbares Gewindeelement, mit welchem die Stange über eine Kopplungsverzahnung in Eingriff steht. Ferner weist die Einstellmechanik ein starr an der Stange befestigtes Verbindungselement auf, welches sich durch das Gewindeelement hindurch erstreckt und an einer der Stange abgewandten Seite aus dem Gewindeelement herausragt. Dieses Verbindungselement erstreckt sich innerhalb des Gewindeelements mit einem radialen Freiraum.

Wie einleitend bereits erläutert, ermöglicht die Einstellmechanik, welche durch wiederholte relative Drehbewegungen zwischen dem Rohr und der Stange betätigbar ist, eine stabile und belastbare Ausbildung des Marknagels, indem insbesondere axiale Druckbelastungen, beispielsweise während eines Stehens, nicht zu einer ungewollten Betätigung der Einstellmechanik führen. Insbesondere kann es vorgesehen sein, dass durch ein Vor- und Zurückdrehen der Stange relativ zu dem Rohr der Abstand zwischen der Stange und dem Rohr um einen vorgegebenen Stellweg vergrößert oder verkleinert wird, so dass die gewünschte Verlängerung oder Verkürzung durch eine klar definierte und bewusst auszuführende zweischrittige Betätigung der Einstellmechanik erreicht werden kann. Im eingesetzten Zustand des Marknagels können die Drehbewegungen durch relatives Verdrehen der über den Marknagel miteinander verbundenen Knochenteile auf die Stange und/oder das Rohr übertragen werden. Die Einstellmechanik kann insbesondere durch den Patienten selbst betätigt werden, wobei beispielsweise eine zuvor genau mit dem Arzt abgesprochene Betätigungsrate auf einfache Weise umgesetzt und von dem Patienten dokumentiert werden kann.

Um die Stange sukzessive in dem Rohr bewegen zu können, steht die Stange mit dem Gewindeelement über eine Kopplungsverzahnung in Eingriff, so dass das Gewindeelement durch eine Drehung der Stange relativ zu dem Rohr axial in dem Rohr bewegt werden kann. Dabei kann das Gewindeelement beispielsweise in einer ersten Drehrichtung von der Stange mitnehmbar sein, während die Stange in einer der ersten Drehrichtung entgegengesetzten zweiten Drehrichtung relativ zu dem Gewindeelement drehbar sein kann, so dass durch Drehen der Stange entlang der ersten Drehrichtung das Gewindeelement axial in dem Rohr bewegt werden und die Einstellmechanik durch darauffolgendes Drehen der Stange entlang der zweiten Drehrichtung wieder in die Ausgangsstellung überführt werden kann. In Abhängigkeit von der Steigung des Innengewindes des Rohres bzw. eines Außengewindes des Gewindeelements kann durch die Betätigung der Einstellmechanik eine Verkürzung des Marknagels oder eine Verlängerung des Marknagels erreicht werden, wobei durch die Gewindesteigung auch die während einer Betätigung der Einstellmechanik zu erreichende Verlängerung oder Verkürzung beeinflussbar ist. Ferner kann der Winkel, um welchen die Stange relativ zu dem Rohr drehbar ist, vorgegeben sein, so dass die axiale Bewegung des Gewindeelements infolge einer Betätigung der Einstellmechanik klar vorbestimmt sein kann.

Um die gewünschte zuverlässige Kopplung zwischen der Stange und dem Rohr zu erreichen, umfasst die Einstellmechanik ferner ein Verbindungselement, welches starr an der Stange befestigt ist. Über dieses Verbindungselement ist die Stange folglich mit der innerhalb des Rohres angeordneten Einstellmechanik gekoppelt, so dass letztlich auch eine Kopplung zwischen der Stange und dem Rohr besteht. Die Stange kann dadurch in dem Rohr gehalten sein und der Marknagel im zusammengesetzten Zustand ein einziges Teil bilden, ohne dass sich die Stange unerwartet aus dem Rohr lösen kann. Dies ermöglicht insbesondere einen komfortablen Einsatz des Marknagels während der Operation, da lediglich ein einziges Teil in den Markkanal eingesetzt werden muss, ohne darauf achten zu müssen, dass sich die Stange und das Rohr nicht voneinander lösen. Zudem kann durch das Verbindungselement eine zuverlässige Übertragung von insbesondere axialen Kräften, die relativ zu dem Rohr auf die Stange ausgeübt werden, auf die Einstellmechanik erreicht werden, um eine zuverlässige Betätigung der Einstellmechanik durch die relativen Drehbewegungen zu ermöglichen.

Bei einer solchen Kopplung zwischen der Einstellmechanik und der gekrümmten Stange besteht grundsätzlich die Problematik, dass das aus der Stange herausragende Verbindungselement während einer Drehung der Stange relativ zu der Drehachse und somit dem Gewindeelement, welches sich geradlinig entlang der Drehachse erstrecken kann, ausgelenkt werden kann. Um eine solche Taumelbewegung des Verbindungselements jedoch zu ermöglichen und eine durch die Auslenkung des Verbindungselements bedingte Übertragung von Biegemomenten auf das Verbindungselement zu verhindern, erstreckt sich das Verbindungselement des hierin offenbarten Marknagels innerhalb des Gewindeelements mit einem radialen Freiraum. Dies ermöglicht es dem Verbindungselement, welches beispielsweise als eine aus der Stange herausragende Schraube ausgebildet sein kann, während einer Drehung der Stange gegenüber der Drehachse des Gewindeelements ausgelenkt zu werden, ohne an einer Innenseite des Gewindeelements anzuschlagen. Der radiale Freiraum kann daher insbesondere derart gewählt sein, dass das Verbindungselement während einer Drehung der Stange relativ zu dem Rohr zum Betätigen der Einstellmechanik nicht an dem Gewindeelement anschlägt. Dementsprechend kann sich das Verbindungselement frei in dem radialen Freiraum bewegen und die Drehung der Stange relativ zu dem Rohr führt nicht zu einer Biegebelastung des Verbindungselements, welche etwa ein Brechen des Verbindungselements zur Folge haben könnte, so dass der Marknagel gekrümmt und dennoch zuverlässig und belastbar bei gleichzeitig bestehender axialer Kopplung zwischen dem Rohr und der Stange ausgebildet werden kann.

Weitere Ausführungsformen sind den abhängigen Ansprüchen, der Beschreibung sowie den Zeichnungen zu entnehmen. Angaben einer radialen oder axialen Richtung im Zusammenhang mit solchen Ausführungsformen beziehen sich grundsätzlich auf die Drehachse des Gewindeelements, sofern keine andere Definition angegeben ist.

Bei einigen Ausführungsformen kann sich ein aus dem Gewindeelement herausragender Teil des Verbindungselements mit einem radialen Freiraum in dem Rohr erstrecken. Insbesondere kann sich der gesamte aus dem Gewindeelement herausragende Teil des Verbindungselements mit einem radialen Freiraum erstrecken, so dass dieser Teil während einer Betätigung der Einstellmechanik relativ zu der Drehachse des Gewindeelements ausgelenkt werden kann. Insbesondere kann der radiale Freiraum in dem Gewindeelement und/oder in dem Rohr derart bemessen sein, dass das Verbindungselement im Zuge einer Betätigung der Einstellmechanik nicht an einer Innenseite des Gewindeelements und/oder des Rohres anschlägt. Der radiale Freiraum für das Verbindungselement kann konstant sein oder sich ausgehend von der Stange zunehmend - stetig oder stufenartig - vergrößern, um die mit dem Abstand zu der Stange zunehmende Auslenkung des Verbindungselements zu ermöglichen.

Das Verbindungselement kann bei einigen Ausführungsformen geradlinig ausgebildet sein. Beispielsweise kann das Verbindungselement mit der Stange verschraubbar sein, um eine Kopplung zwischen der Einstellmechanik und der Stange herzustellen. Das Verbindungselement kann somit insbesondere als eine Schraube mit einem Schaft und einem Schraubenkopf ausgebildet sein, wobei der Schaft in der Stange verschraubt und der Schraubenkopf an einem aus dem Gewindeelement herausragenden Teil der Schraube ausgebildet sein kann. Alternativ dazu kann das Verbindungselement auch form- und/oder kraftschlüssig mit der Stange verbunden und beispielsweise in der Stange verstemmt oder in die Stange hineingepresst sein. Insbesondere kann sich das Verbindungselement geradlinig entlang der Drehachse des Gewindeelements erstrecken, wenn eine Betätigung der Einstellmechanik abgeschlossen ist und sich die Einstellmechanik und/oder die Stange in einer Ausgangsstellung befinden.

Bei einigen Ausführungsformen kann das Gewindeelement an einem der Stange zugewandten Endabschnitt des Gewindeelements mittels des Gewindeelements zentriert sein. Insbesondere kann sich das Verbindungselement dazu in dem der Stange zugewandten Endabschnitt des Gewindeelements nicht mit einem radialen Freiraum erstrecken, sondern von dem Gewindeelement zumindest abschnittsweise radial spielreduziert umschlossen sein.

Indem das Verbindungselement an dem Endabschnitt mittels des Gewindeelements des Gewindeelements zentriert sein kann, kann auch die Stange relativ zu dem Gewindeelement zentriert werden, um einen sicheren und vollständigen Eingriff zwischen der Stange und dem Gewindeelement über die Kopplungsverzahnung sicherzustellen. Durch die Zentrierung kann insbesondere verhindert werden, dass sich die Stange im Zuge einer Betätigung der Einstellmechanik radial relativ zu dem Gewindeelement bewegt, wodurch die Kopplungsverzahnung beispielsweise nach der Betätigung der Einstellmechanik nicht wieder vollständig in Eingriff gelangen könnte.

Beispielsweise kann es vorgesehen sein, dass die Stange das Gewindeelement entlang einer ersten Drehrichtung um die Drehachse über die Kopplungsverzahnung mitnimmt, wohingegen die Kopplungsverzahnung in einer der ersten Drehrichtung entgegengesetzten zweiten Drehrichtung einen Freilauf aufweist, so dass die Stange relativ zu dem Gewindeelement verdreht und die Einstellmechanik wieder in die Ausgangsstellung überführt werden kann. Um einen solchen Freilauf zu ermöglichen, muss die Kopplungsverzahnung jedoch bei einer Drehung der Stange entlang der zweiten Drehrichtung kurzzeitig außer Eingriff geraten, so dass eine an der Stange ausgebildete Verzahnung einen Zahn an einer an dem Gewindeelement ausgebildeten Verzahnung überspringen kann. Insbesondere in dem Moment des Überspringens besteht somit keine radiale Kopplung zwischen der Stange und dem Gewindeelement über die Kopplungsverzahnung, so dass eine radiale Bewegung der Stange relativ zu dem Gewindeelement grundsätzlich möglich wäre. Dies kann jedoch durch die Zentrierung des Verbindungselements in dem Endabschnitt des Gewindeelements zuverlässig verhindert werden.

Indem die Zentrierung des Verbindungselements ferner in oder an einem Endabschnitt des Gewindeelements erfolgt, kann das Verbindungselement lediglich unmittelbar in einem sich an die Stange anschließenden Abschnitt von dem Gewindeelement umschlossen sein. In dem Endabschnitt des Gewindeelements wird das Verbindungselement während einer Drehung der Stange aufgrund des lediglich geringen Abstands zu der Stange jedoch allenfalls minimal relativ zu der Drehachse des Gewindeelements ausgelenkt, so dass durch eine radial spielreduzierte Führung des Verbindungselements in dem Endabschnitt allenfalls geringfügige und daher tolerierbare Biegemomente, die lediglich aus der Zentrierung entstehen, auf das Verbindungselement übertragen werden können. Der weiter aus der Stange heraus und durch das Gewindeelement hindurchragende Teil des Verbindungselements, bei welchem die Taumelbewegung eine größere radiale Auslenkung relativ zu der Drehachse des Gewindeelements bedingt, kann hingegen durch den radialen Freiraum ermöglicht werden, so dass die Übertragung relevanter oder kritischer Biegemomente auf das Verbindungselement zuverlässig vermieden werden kann.

Bei einigen Ausführungsformen kann das Gewindeelement an dem der Stange zugewandten Endabschnitt einen Zentrierabschnitt für das Verbindungselement aufweisen. Ein solcher Zentrierabschnitt kann beispielsweise als eine innen an dem Gewindeelement umlaufende Ringfläche ausgebildet sein, durch welche ein Innendurchmesser des Gewindeelements im Bereich des Endabschnitts verringert ist. Alternativ zu einem solchen umlaufenden Steg kann das Gewindeelement beispielsweise auch mehrere, insbesondere zwei, vier, sechs oder acht radial nach innen gerichtete Zentrierstützen aufweisen, welche eine radiale Auslenkung des Verbindungselements innerhalb des Endabschnitts des Gewindeelements relativ zu dem Gewindeelement einschränken und/oder im Wesentlichen verhindern.

Ferner kann das Gewindeelement bei einigen Ausführungsformen zum Zentrieren des Verbindungselements einen radial nach innen ragenden Zentrierabschnitt aufweisen, dessen axiale Länge in einem Bereich von einem Zehntel bis zu einem Drittel, insbesondere in einem Bereich von einem Achtel bis zu einem Viertel, der axialen Länge des Gewindeelements liegt. Die axiale Länge des Zentrierabschnitts des Gewindeelements kann bei einigen Ausführungsformen zudem etwa ein Siebtel der axialen Länge des Gewindeelements betragen. Der Zentrierabschnitt kann beispielsweise als ein an einer Innenseite des Gewindeelements umlaufender Steg ausgebildet sein oder mehrere radial nach innen ragende Zentrierstützen aufweisen.

Der Zentrierabschnitt des Gewindeelements kann somit einen axial schmalen, insbesondere ringartigen, Abschnitt an einem der Stange zugewandten Ende des Gewindeelements bilden, in welchem eine radiale Auslenkung des Verbindungselements relativ zu dem Gewindeelement eingeschränkt und/oder blockiert sein kann. Dadurch kann, wie bereits erläutert, eine radiale Relativbewegung zwischen der Stange und dem Gewindeelement zuverlässig vermieden werden, um die Kopplungsverzahnung insbesondere zum Ende einer Betätigung der Einstellmechanik zuverlässig wieder in Eingriff zu bringen, ohne jedoch die Auslenkung weiter von der Stange entfernter Abschnitte des Verbindungselements einzuschränken.

Das Verbindungselement kann sich bei einigen Ausführungsformen zwischen dem der Stange zugewandten Endabschnitt des Gewindeelements und einer der Stange abgewandten Stirnseite des Gewindeelements vollständig mit einem radialen Freiraum in dem Gewindeelement erstrecken.

Insbesondere kann vorgesehen sein, dass das Verbindungselement ausschließlich an dem der Stange zugewandten Endabschnitt des Gewindeelements zentriert und/oder radial spielreduziert umschlossen ist, während sich das Verbindungselement davon abgesehen über seine gesamte axiale Länge mit einem radialen Freiraum in dem Gewindeelement erstrecken kann. Insbesondere kann durch diese Zentrierung des Verbindungselements der Gegebenheit Rechnung getragen werden, dass sich das Ausmaß der radialen Auslenkungen des Verbindungselements relativ zu der Drehachse während einer Taumelbewegung mit zunehmendem Abstand von der Stange erhöht. Solche relativ großen Auslenkungen können jedoch dadurch ermöglicht werden, dass sich das Verbindungselement außerhalb des Endabschnitts des Gewindeelements mit dem radialen Freiraum erstreckt und dementsprechend dort radial nicht geführt ist. Gleichzeitig kann durch die Zentrierung des Verbindungselements in dem Endabschnitt auch eine Zentrierung der Kopplungsverzahnung erreicht werden, wobei das Verbindungselement während der Taumelbewegung aufgrund der Nähe zu der Stange in dem Endabschnitt jedoch keine Auslenkungen erfährt, die zu großen Biegemomenten und einer Beschädigung des Verbindungselements führen könnten.

Das Verbindungselement kann bei einigen Ausführungsformen eine radiale Anschlagsverbreiterung aufweisen, wobei der Endabschnitt des Gewindeelements und die Anschlagsverbreiterung des Verbindungselements eine axiale Relativbewegung zwischen der Stange und dem Gewindeelement begrenzen können.

Insbesondere können die radiale Anschlagsverbreiterung des Verbindungselements und der Endabschnitt des Gewindeelements, an welchem das Verbindungselement zentriert ist, derart zusammenwirken, dass eine axiale Relativbewegung zwischen der Stange und dem Gewindeelement, durch welche die Stange aus dem Rohr herausbewegbar ist, begrenzt ist. Durch eine solche Begrenzung kann somit auch eine axiale Relativbewegung zwischen der Stange und dem Rohr eingeschränkt werden, so dass ein Lösen der Stange aus dem Rohr, insbesondere während des Einsetzens des Marknagels während einer Operation, zuverlässig verhindert werden kann. Eine axiale Bewegung der Stange in das Rohr hinein kann ferner durch das Gewindeelement und insbesondere die Kopplungsverzahnung begrenzt sein, so dass mittels der Anschlagsverbreiterung des Verbindungselements einerseits und der Kopplungsverzahnung andererseits axiale Relativbewegungen zwischen der Stange und dem Rohr in beiden axialen Richtungen eingeschränkt sein können. Es kann jedoch auch vorgesehen sein, dass an dem Gewindeelement ein dem Endabschnitt entgegengesetzter Blockierabschnitt ausgebildet ist, an welchem die Anschlagsverbreiterung des Verbindungselements bei einer Bewegung der Stange in das Rohr hinein anschlägt, um eine solche axiale Relativbewegung zwischen der Stange und dem Rohr zusätzlich zu begrenzen.

Insbesondere können die radiale Anschlagsverbreiterung und das Gewindeelement derart aufeinander abgestimmt sein, dass die Anschlagsverbreiterung während einer Drehung der Stange zum Betätigen der Einstellmechanik und der daraus folgenden Taumelbewegung des Verbindungselements nicht an einer Innenseite des Gewindeelements anschlägt. Folglich kann für das Verbindungselement auch im Bereich der radialen Anschlagsverbreiterung ein für die Taumelbewegung ausreichender radialer Freiraum in dem Gewindeelement zur Verfügung stehen, so dass die radiale Anschlagsverbreiterung einer Auslenkung des Verbindungselements gegenüber dem Gewindeelement bei solchen Ausführungsformen nicht entgegensteht, die Stange jedoch in dem Rohr gegen ein ungewolltes Lösen gesichert ist. Während die Anordnung des Verbindungselements in dem Gewindeelement mit einem radialen Freiraum somit eine gekrümmte Ausbildung des Marknagels ermöglicht, ohne dass während des Betätigens der Einstellmechanik Biegemomente auf das Verbindungselement übertragen werden, kann durch die Anschlagsverbreiterung eine zuverlässige axiale Kopplung zwischen der Stange und dem Rohr erreicht werden, um die bei einer Entkopplung zwischen der Stange und der Einstellmechanik bestehende Problematik eines ungewollten Lösens der Stange aus dem Rohr zu überwinden. Insofern bieten solche Ausführungsformen eine einfache Möglichkeit, eine zuverlässige Kopplung zwischen der Stange und dem Rohr bei einem gekrümmten Marknagel zu erreichen.

Ferner kann es kann bei einigen Ausführungsformen vorgesehen sein, dass eine Drehung der Stange um die Drehachse entlang einer ersten Drehrichtung über die Kopplungsverzahnung auf das Gewindeelement übertragbar ist. Wie bereits erläutert, kann durch eine solche Übertragung der Drehung auf das Gewindeelement das Gewindeelement axial in dem Rohr bewegt werden, so dass auch die Stange durch das axiale Ineinandergreifen der Kopplungsverzahnung nach der Drehung des Gewindeelements axial relativ zu dem Rohr bewegt sein kann. In Abhängigkeit von der Gewindesteigung kann die Stange dabei aus dem Rohr heraus oder in das Rohr hinein bewegbar sein, so dass der Marknagel als ein Distraktionsmarknagel oder als ein Kontraktionsmarknagel ausgebildet sein kann.

Bei einigen Ausführungsformen kann die Stange in einer der ersten Drehrichtung entgegengesetzten zweiten Drehrichtung relativ zu dem Gewindeelement um die Drehachse drehbar sein. Wenn die Stange entlang der zweiten Drehrichtung um die Drehachse gedreht wird, wird das Gewindeelement folglich bei solchen Ausführungsformen nicht mitgenommen, so dass das Gewindeelement axial in dem Rohr unbewegt bleibt und bei einer Drehung der Stange entlang der zweiten Drehrichtung keine Längenveränderung des Marknagels erfolgt. Vielmehr kann die Einstellmechanik durch Drehen der Stange entlang der zweiten Drehrichtung wieder in eine Ausgangsstellung überführt werden, um eine neuerliche Längenveränderung durch darauffolgendes Drehen entlang der ersten Drehrichtung zu ermöglichen.

Eine Betätigung der Einstellmechanik zur Veränderung der Länge des Marknagels um einen vorbestimmten Stellweg kann somit zweischrittig erfolgen und zunächst eine Drehung der Stange entlang der ersten Drehrichtung umfassen, wobei durch diese Drehung die Längenveränderung erfolgt. Daraufhin kann die Betätigung der Einstellmechanik durch eine Drehung der Stange entlang der zweiten Drehrichtung abgeschlossen werden, um die Einstellmechanik wieder in die Ausgangsstellung zu überführen. Insbesondere kann die Kopplungsverzahnung dazu in der zweiten Drehrichtung eine Freilaufrichtung aufweisen, wobei während des Drehens der Stange entlang der zweiten Drehrichtung eine an der Stange ausgebildete Verzahnung insbesondere von einer an dem Gewindeelement ausgebildeten Verzahnung axial entfernt werden kann, um die Verzahnung der Stange relativ zu der Verzahnung des Gewindeelements um einen Zahn zu verdrehen. In der ersten Drehrichtung kann die Kopplungsverzahnung hingegen eine Sperre bilden, so dass die Drehung der Stange auf das Gewindeelement übertragen werden kann. Der Freilauf und die Sperre können beispielsweise durch geeignete Steigungen der Verzahnungen der Stange und des Gewindeelements, welche die Kopplungsverzahnung bilden, erreicht werden.

Bei einigen Ausführungsformen kann eine Drehbewegung der Stange um die Drehachse relativ zu dem Rohr begrenzt sein. Insbesondere können Drehungen der Stange relativ zu dem Rohr sowohl in der ersten Drehrichtung als auch in der zweiten Drehrichtung begrenzt sein, so dass eine Betätigung der Einstellmechanik durch Drehungen der Stange um klar definierte Winkel erfolgen kann. Insbesondere kann durch eine Drehung der Stange um den definierten Winkel entlang der ersten Drehrichtung eine vorgegebene axiale Längenveränderung erreicht werden, um die Einstellmechanik durch Zurückdrehen der Stange um denselben Winkel entlang der zweiten Drehrichtung wieder in die Ausgangsstellung zu versetzen. Die Stange kann während des Betätigens der Einstellmechanik beispielsweise um einen derartigen Winkel verdrehbar sein, dass eine Verzahnung der Stange beim Zurückdrehen der Stange entlang der zweiten Drehrichtung gerade um einen Zahn relativ zu einer Verzahnung des Gewindeelements, welche gemeinsam die Kopplungsverzahnung bilden, zurückgedreht wird.

Die Stange kann bei einigen Ausführungsformen eine Passfeder aufweisen, welche innerhalb des Rohres in einer Nut geführt ist, wobei die Nut die Drehbewegung der Stange um die Drehachse begrenzen kann. Die Nut kann einen Anschlag für die Passfeder der Stange bilden, so dass durch die Breite der Passfeder und die Breite der Nut vorgegeben werden kann, um welchen Winkel die Stange relativ zu dem Rohr verdrehbar ist. Alternativ dazu kann es auch vorgesehen sein, dass in einem Endbereich des Rohres, aus welchem die Stange herausragt, eine Passfeder vorgesehen ist, welche mit einer an der Stange ausgebildeten Nut zusammenwirkt, um eine Drehbewegung der Stange um die Drehachse zu begrenzen. Durch eine solche Anordnung der Passfeder kann die Passfeder in einem Bereich des Rohres angebracht sein, welcher nicht für eine Drehung des Gewindeelements freigehalten werden muss.

Das Gewindeelement kann bei einigen Ausführungsformen an einer der Stange abgewandten Seite über eine Blockierverzahnung mit einem Blockierelement in Eingriff stehen, wobei das Verbindungselement durch das Blockierelement hindurchgeführt sein und mit einem Verlängerungsabschnitt an einer dem Gewindeelement abgewandten Seite aus dem Blockierelement herausragen kann. Das Verbindungselement kann sich ferner mit einem radialen Freiraum in dem Blockierelement erstrecken.

Insbesondere kann sich das Blockierelement somit axial an einer der Stange entgegengesetzten Seite an das Gewindeelement anschließen und das Verbindungselement kann sich ausgehend von der Stange durch das Gewindeelement und das Blockierelement hindurch erstrecken. Indem sich das Verbindungselement dabei auch in dem Blockierelement mit einem radialen Freiraum erstrecken kann, kann das Verbindungselement während einer Drehung der Stange zum Betätigen der Einstellmechanik in dem Blockierelement radial zu der Drehachse des Gewindeelements ausgelenkt werden, ohne dass Biegemomente auf das Verbindungselement übertragen werden. Wiederum kann der radiale Freiraum an dem Blockierelement insbesondere derart gewählt sein, dass das Verbindungselement innerhalb des Blockierelements während einer Betätigung der Einstellmechanik radial nicht anschlägt. Der radiale Freiraum in dem Blockierelement kann dem radialen Freiraum in dem Gewindeelement entsprechen oder größer als der radiale Freiraum in dem Gewindeelement sein, um die mit dem Abstand von der Stange größer werdende radiale Auslenkung des Verbindungselements während eines Betätigens der Einstellmechanik zu ermöglichen.

Darüber hinaus kann vorgesehen sein, dass sich auch der Verlängerungsabschnitt mit einem radialen Freiraum in dem Rohr erstreckt, so dass auch der Verlängerungsabschnitt radial während einer Drehung der Stange ausgelenkt werden kann. Über den Verlängerungsabschnitt kann zudem eine Kopplung zwischen der Stange und dem Blockierelement erreicht werden, wie nachstehend noch näher erläutert ist.

Das Blockierelement kann bei einigen Ausführungsformen verdrehsicher in dem Rohr geführt sein. Insbesondere kann das Blockierelement eine Passfeder aufweisen, welche innerhalb des Rohres in einer Nut geführt ist.

Das Blockierelement kann somit insbesondere axial in dem Rohr verschiebbar, jedoch nicht um die Drehachse drehbar sein. Beispielsweise kann dazu die bereits genannte Nut, innerhalb welcher eine Passfeder der Stange zum Begrenzen von Drehbewegungen der Stange geführt sein kann, vorgesehen sein, wobei das Blockierelement eine im Wesentlichen spielfrei in der Nut geführte Passfeder aufweisen kann, um Drehungen des Blockierelements zu verhindern. Insbesondere kann die Passfeder des Blockierelements somit dicker als die Passfeder der Stange ausgebildet sein, so dass die Stange um einen vorgegebenen Winkel in der Nut drehbar, das Blockierelement jedoch verdrehsicher in der Nut geführt sein kann.

Bei einigen Ausführungsformen kann die Blockierverzahnung in der ersten Drehrichtung einen Freilauf aufweisen, durch welchen das Gewindeelement für eine Drehung entlang der ersten Drehrichtung relativ zu dem Blockierelement freigegeben ist. In der zweiten Drehrichtung kann die Blockierverzahnung hingegen eine Sperre bilden, welche eine Drehung des Gewindeelements relativ zu dem Blockierelement entlang der zweiten Drehrichtung blockiert.

Insbesondere kann es der Freilauf in der ersten Drehrichtung ermöglichen, über die Stange eine Drehung auf das Gewindeelement zu übertragen, um das Gewindeelement axial in dem Rohr zu bewegen und dadurch eine Längenveränderung des Marknagels zu erreichen. Durch das Blockieren einer Drehung des Gewindeelements entlang der zweiten Drehrichtung kann ferner erreicht werden, dass eine Drehung der Stange entlang der zweiten Drehrichtung nicht auf das Gewindeelement übertragen wird, sondern die Stange relativ zu dem Gewindeelement verdreht werden kann. Dadurch kann die Einstellmechanik wieder in eine Ausgangsstellung überführt werden, um ein kontrolliertes und schrittweises Verändern der Länge des Marknagels zu erreichen.

Der Freilauf und die Sperre der Blockierverzahnung können insbesondere durch eine unterschiedliche Ausbildung von Verzahnungen an dem Gewindeelement und Verzahnungen an dem Blockierelement, welche die Blockierverzahnung bilden, in den beiden Drehrichtungen erreicht werden. Beispielsweise können die Verzahnungen blockierend und/oder senkrecht zu der zweiten Drehrichtung, jedoch nur leicht ansteigend zu der ersten Drehrichtung ausgerichtet sein. Dadurch kann das Gewindeelement beispielsweise bei einer Drehung in der ersten Drehrichtung über die Verzahnung des Blockierelements entlanggleiten und sich axial von dem Blockierelement entfernen, so dass eine Verzahnung des Gewindeelements einen Zahn einer Verzahnung des Blockierelements überspringen kann. Hingegen kann eine Drehung entlang der zweiten Drehrichtung durch die Blockierverzahnung blockiert werden. In ähnlicher Weise können auch die Verzahnungen der Kopplungsverzahnung ausgebildet sein, um ein Mitnehmen des Gewindeelements in der ersten Drehrichtung und eine Drehung der Stange relativ zu dem Gewindeelement in der zweiten Drehrichtung zu ermöglichen.

Ferner kann die Einstellmechanik bei einigen Ausführungsformen ein Zentrierelement umfassen, welches das Blockierelement gegen Bewegungen in radialer Richtung relativ zu dem Gewindeelement blockiert. Insbesondere kann das Gewindeelement bei einer Drehung entlang der ersten Drehrichtung relativ zu dem Blockierelement axial außer Eingriff zu dem Blockierelement gelangen, indem eine an dem Gewindeelement ausgebildete Verzahnung relativ zu einer an dem Blockierelement ausgebildeten Verzahnung der Blockierverzahnung um einen Zahn verdreht wird. Durch das Zentrierelement können radiale Relativbewegungen zwischen dem Blockierelement und dem Gewindeelement während einer solchen Drehung verhindert werden, so dass die Blockierverzahnung daraufhin wieder zuverlässig in Eingriff gelangen kann.

Das Zentrierelement kann bei einigen Ausführungsformen als eine Zentrierhülse ausgebildet sein, auf oder in die das Blockierelement und das Gewindeelement steckbar sind. Durch eine solche Zentrierhülse können das Blockierelement und das Gewindeelement radial miteinander verbunden sein, so dass eine in radialer Richtung beispielsweise auf das Blockierelement wirkende Kraft über die Zentrierhülse auf das Gewindeelement und über das Gewindeelement auf das Rohr übertragen werden kann. Radiale Relativbewegungen zwischen dem Blockierelement und dem Gewindeelement können dadurch verhindert werden, um insbesondere ein sicheres Ineinandergreifen der Verzahnungen der Blockierverzahnung nach einer Drehung des Gewindeelements zu erreichen. Axiale Relativbewegungen zwischen dem Gewindeelement und dem Blockierelement können hingegen möglich sein, indem das Gewindeelement und das Blockierelement axial relativ zu der Zentrierhülse bewegbar sein können.

Die Zentrierhülse kann insbesondere an einer Innenseite oder an einer Außenseite des Gewindeelements und des Blockierelements angeordnet sein, um die axiale Verbindung zwischen dem Gewindeelement und dem Blockierelement herzustellen, so dass die Blockierverzahnung insbesondere sich radial innen oder radial außen an die Zentrierhülse anschließend ausgebildet sein kann. Insbesondere bei einer Anordnung der Zentrierhülse an einer Innenseite des Gewindeelements und des Blockierelements kann die Zentrierhülse ausreichend dünn ausgebildet sein, um dennoch eine Anordnung des Verbindungselements mit einem radialen Freiraum auch im Bereich der Zentrierhülse erreichen zu können.

Bei einigen Ausführungsformen können das Blockierelement und das Gewindeelement an ihrer Innenseite oder ihrer Außenseite eine jeweilige radiale Freistellung aufweisen, wobei das Zentrierelement in den radialen Freistellungen angeordnet sein und eine radiale Verbindung zwischen dem Gewindeelement und dem Blockierelement bilden kann. Insbesondere kann durch solche radiale Freistellungen erreicht werden, dass das Zentrierelement und insbesondere eine Zentrierhülse an der Innenseite des Gewindeelements und des Blockierelements angeordnet werden kann, ohne den radialen Freiraum für das Verbindungselement zu begrenzen. Eine Anordnung des Zentrierelements an den Innenseiten des Gewindeelements und des Blockierelements ermöglicht es zudem, die Kopplungsverzahnung radial außen von dem Zentrierelement anzuordnen, so dass die Verzahnungen der Blockierverzahnung möglichst groß und zuverlässig ausgebildet werden können.

Die Kopplungsverzahnung kann bei einigen Ausführungsformen in der zweiten Drehrichtung einen Freilauf aufweisen, welcher eine Drehung der Stange relativ zu dem durch die Blockierverzahnung blockierten Gewindeelement entlang der zweiten Drehrichtung ermöglicht. Wie bereits erläutert, kann die Einstellmechanik durch eine solche Drehung der Stange in der zweiten Drehrichtung wieder in eine Ausgangsstellung überführt werden, ohne dass der Marknagel eine Längenänderung erfährt. Dies kann durch eine geeignete Ausbildung der Verzahnungen der Stange und des Gewindeelements, welche gemeinsam die Kopplungsverzahnung bilden, erreicht werden.

Bei einigen Ausführungsformen kann das Blockierelement über das Verbindungselement in Richtung des Gewindeelements vorgespannt sein. Durch eine solche Vorspannung kann insbesondere eine Kraft zum Schließen der Blockierverzahnung auf das Blockierelement übertragen werden, um das Gewindeelement beispielsweise zuverlässig gegen eine Drehung entlang der zweiten Drehrichtung blockieren zu können. Zudem kann durch die Vorspannung des Blockierelements in Richtung des Gewindeelements auch die Stange, an welcher das Verbindungselement starr befestigt ist, in Richtung des Gewindeelements vorgespannt sein, um die Kopplungsverzahnung in einem unbetätigten Zustand der Einstellmechanik zu schließen. Die Kopplungsverzahnung kann jedoch grundsätzlich auch bereits durch die vom menschlichen Körpergewebe hervorgerufene Kraft auf die beiden Knochenteile, an welchen die Stange und das Rohr befestigt sind, zuverlässig in Eingriff gehalten werden.

Ferner kann das Blockierelement bei einigen Ausführungsformen entgegen der Rückstellkraft der Vorspannung außer Eingriff mit dem Gewindeelement bringbar sein. Dadurch kann insbesondere das Gewindeelement in der ersten Drehrichtung relativ zu dem Blockierelement drehbar sein, um eine Veränderung der Länge des Marknagels zu erreichen. Beispielsweise kann das Blockierelement bei einem Übertragen eines Drehmoments auf das Gewindeelement entlang der ersten Drehrichtung durch die aneinander gleitenden Verzahnungen der Blockierverzahnung axial entgegen der Vorspannung zurückgedrängt werden, um eine Drehung des Gewindeelements zuzulassen. Sobald die Drehung des Gewindeelements jedoch abgeschlossen ist, können die Verzahnungen der Blockierverzahnung aufgrund der Vorspannung des Blockierelements wieder in Eingriff gelangen.

Mit dem Verbindungselement kann bei einigen Ausführungsformen eine Spanneinrichtung gekoppelt sein, welche das Blockierelement in Richtung des Gewindeelements vorspannt. Insbesondere kann mit dem Verbindungselement eine Feder gekoppelt sein, welche das Blockierelement in Richtung des Gewindeelements vorspannt. Über diese Spanneinrichtung kann somit letztlich auch die Stange, an welcher das Verbindungselement starr befestigt ist, mit dem Blockierelement gekoppelt sein, so dass insbesondere axial auf die Stange wirkende Kräfte auf das Blockierelement übertragen werden können. Insbesondere kann dadurch bei einem axialen Entfernen der Stange von dem Gewindeelement und dem Blockierelement bei einem Zurückdrehen der Stange entlang der zweiten Drehrichtung, bei welchem eine Verzahnung der Stange um einen Zahn relativ zu einer Verzahnung des Gewindeelements zurückgesetzt werden kann, über das Verbindungselement und die Spanneinrichtung eine axiale Kraft auf das Blockierelement übertragen werden, um die Blockierverzahnung zuverlässig zu schließen und eine Drehung des Gewindeelements zu verhindern.

Die Spanneinrichtung kann bei einigen Ausführungsformen eine Feder umfassen oder als eine Feder ausgebildet sein, welche an dem Blockierelement und an dem Verlängerungsabschnitt des Verbindungselements abgestützt ist. Beispielsweise kann das Verbindungselement dazu als eine Schraube mit einem Schraubenkopf ausgebildet sein, wobei eine dem Blockierelement zugewandte Unterseite des Schraubenkopfes die Abstützung der Feder bilden kann.

Bei einigen Ausführungsformen kann die Feder an einer dem Gewindeelement abgewandten Stirnseite des Blockierelements abgestützt sein. Insbesondere kann die Feder zwischen einem dem Blockierelement zugewandten Schraubenkopf eines als Schraube ausgebildeten Verbindungselements und einer Stirnseite des Blockierelements angeordnet sein, so dass die Feder sich axial zwischen dem Blockierelement und einer Unterseite des Schraubenkopfs erstrecken kann.

Alternativ dazu kann das Blockierelement bei einigen Ausführungsformen eine Federaufnahme aufweisen, in welcher die Feder zumindest teilweise aufgenommen ist, und die Feder kann in Richtung des Gewindeelements an einem Boden der Federaufnahme abgestützt sein. Beispielsweise kann eine solche Federaufnahme als eine Bohrung, ein Schlitz oder eine umlaufende Freistellung in dem Blockierelement ausgebildet sein. Bei solchen Ausführungsformen kann insbesondere eine axiale Länge des aus dem Blockierelement herausragenden Verlängerungsabschnitts des Verbindungselements verkürzt werden, indem ein Teil der Feder axial in dem Blockierelement angeordnet wird. Dadurch kann auch eine maximale radiale Auslenkung des Verbindungselements relativ zu der Drehachse des Gewindeelements während einer Drehung der Stange reduziert werden.

Die Einstellmechanik kann bei einigen Ausführungsformen ferner einen Anschlag aufweisen, welcher eine axiale Bewegung des Verbindungselements entgegen der Kraft der Spanneinrichtung begrenzt.

Insbesondere kann der Anschlag der Einstellmechanik durch ein an dem Verbindungselement ausgebildetes Anschlagselement und ein an einer weiteren Komponente der Einstellmechanik ausgebildetes Anschlagselement gebildet sein, die zusammenwirken, um eine axiale Bewegung des Verbindungselements entgegen der Kraft der Spanneinrichtung, insbesondere einer Feder, zu begrenzen. Entsprechend können - aufgrund der starren Befestigung des Verbindungselements an der Stange - durch den Anschlag auch axiale Relativbewegungen zwischen der Stange und dem Rohr begrenzt werden, um eine Überbelastung der Spanneinrichtung infolge solcher Relativbewegungen zu verhindern. Beispielsweise kann während einer Operation an dem Rohr oder der Stange gezogen werden, um den Marknagel beispielsweise nochmals aus einem Röhrenknochen zu lösen, wobei das Rohr und die Stange relativ zueinander bewegt und die Spanneinrichtung gespannt werden kann. Durch den Anschlag können solche Relativbewegungen jedoch derart begrenzt werden, dass die Spanneinrichtung nicht überlastet wird.

Der Anschlag kann bei einigen Ausführungsformen eine radiale Verbreiterung des Verbindungselements umfassen. Insbesondere kann eine solche radiale Verbreiterung des Verbindungselements bei einer axialen Bewegung des Verbindungselements an einem weiteren Element der Einstellmechanik anschlagen und die Bewegung des Verbindungselements dadurch begrenzen.

Beispielsweise kann die radiale Verbreiterung des Verbindungselements bei einigen Ausführungsformen durch eine Anschlagsverbreiterung gebildet sein, welche an einem Abschnitt des Verbindungselements angeordnet ist, der sich innerhalb des Gewindeelements erstreckt. Dabei können die Anschlagsverbreiterung und ein radial nach innen gerichteter Zentrierabschnitt des Gewindeelements den Anschlag der Einstellmechanik bilden. Bei diesem Zentrierabschnitt des Gewindeelements kann es sich insbesondere um den bereits genannten Zentrierabschnitt handeln, welcher an einem der Stange zugewandten Endabschnitt des Gewindeelements ausgebildet sein kann, um eine radiale Relativbewegung zwischen dem Gewindeelement und der Stange zu begrenzen. Dieser Zentrierabschnitt kann somit eine Doppelfunktion erfüllen und einerseits die Kopplungsverzahnung zentrieren, andererseits jedoch gemeinsam mit der Anschlagsverbreiterung eine axiale Bewegung des Verbindungselements entgegen der Kraft der Spanneinrichtung und somit eine Überbelastung der Spanneinrichtung verhindern. Darüber hinaus kann, wie bereits erläutert, durch das Zusammenwirken des Zentrierabschnitts und der Anschlagsverbreiterung auch eine zuverlässige axiale Kopplung zwischen der Stange und dem Rohr bei gekrümmter Ausbildung des Marknagels erreicht werden.

Alternativ dazu kann bei einigen Ausführungsformen die radiale Verbreiterung des Verbindungselements an dem Verlängerungsabschnitt des Verbindungselements ausgebildet sein, wobei die radiale Verbreiterung und eine dem Gewindeelement abgewandte Stirnseite des Blockierelements den Anschlag der Einstellmechanik bilden können. Insbesondere kann bei solchen Ausführungsformen an dem Blockierelement eine Federaufnahme ausgebildet sein, in welcher eine die Spanneinrichtung bildende Feder teilweise aufgenommen ist. Ferner kann die radiale Verbreiterung des Verbindungselements bei solchen Ausführungsformen insbesondere von einem Schraubenkopf gebildet sein, welcher infolge einer axialen Bewegung des als Schraube ausgebildeten Verbindungselements an dem Blockierelement anschlagen kann. Auch dadurch kann grundsätzlich sowohl eine Überbelastung der Spanneinrichtung als auch ein Lösen der Stange aus dem Rohr verhindert werden.

Bei einigen Ausführungsformen kann das Verbindungselement als Schraube mit einem Schaft und mit einem Schraubenkopf ausgebildet sein, wobei die radiale Verbreiterung von dem Schraubenkopf gebildet ist oder wobei die radiale Verbreiterung zwischen dem Schraubenkopf und dem Blockierelement an dem Schaft der Schraube ausgebildet ist. Auch bei solchen Ausführungsformen kann der Anschlag somit eine Stirnseite des Blockierelements umfassen, welche entweder mit dem Schraubenkopf oder einer eigens dazu vorgesehenen radialen Verbreiterung an dem Schaft der Schraube zusammenwirken kann.

Die Erfindung wird im Folgenden rein beispielhaft anhand von Ausführungsbeispielen unter Bezugnahme auf die Zeichnungen erläutert.

Es zeigen:
- Fig. 1: eine schematische Längsschnittdarstellung eines gekrümmten Marknagels, der ein gekrümmtes Rohr und eine innerhalb des Rohres angeordnete gekrümmte Stange aufweist,
- Fig. 2A und 2B: eine schematische Längsschnittdarstellung und eine schematische perspektivische Längsschnittdarstellung des Marknagels zur Veranschaulichung einer durch wiederholte relative Drehbewegungen zwischen der Stange und dem Rohr betätigbaren Einstellmechanik, mittels derer die Stange relativ zu dem Rohr axial bewegbar ist,
- Fig. 3: eine schematische Längsschnittdarstellung zur näheren Veranschaulichung der Einstellmechanik, und
- Fig. 4: eine schematische Längsschnittdarstellung zur Veranschaulichung der Einstellmechanik einer weiteren Ausführungsform des Marknagels.

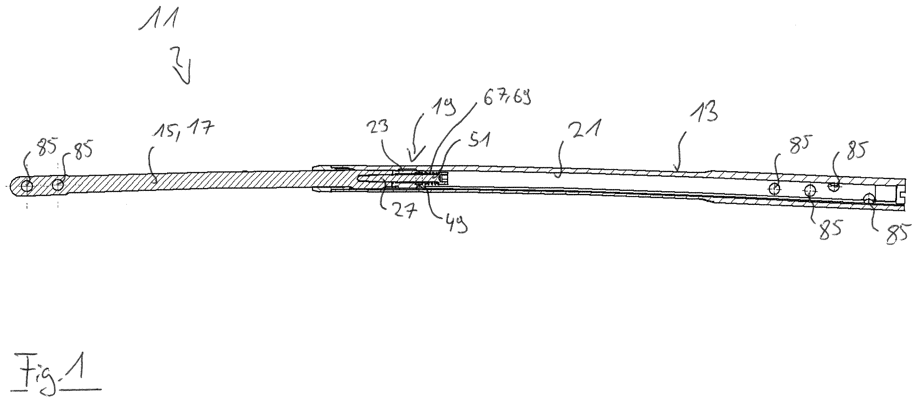

Fig. 1 zeigt einen Marknagel 11, welcher ein gekrümmtes Rohr 13 aufweist, innerhalb dessen eine gekrümmte Stange 15 angeordnet ist. Der Marknagel 11 ist dazu vorgesehen, mit zwei nicht gezeigten Knochenteilen eines durchtrennten Knochens verbunden zu werden und eine zwischen den Knochenteilen liegende Knochenlücke zu überbrücken, so dass die Knochenteile durch sich in der Knochenlücke kontinuierlich neu bildende Knochensubstanz zusammenwachsen können. Dazu sind an dem Rohr 13 mehrere Befestigungsöffnungen 85 vorgesehen, so dass das Rohr 13 beispielsweise an einem ersten Knochenteil befestigt und insbesondere verschraubt werden kann. Die Stange 15 ragt an einem den Befestigungsöffnungen 85 des Rohres 13 entgegengesetzten Ende des Rohres 13 mit einem Befestigungsabschnitt 17 aus dem Rohr 13 heraus, an welchem ebenfalls mehrere Befestigungsöffnungen 85 ausgebildet sind, um die Stange 15 an einem nicht gezeigten zweiten Knochenteil befestigen zu können.

Aufgrund der Krümmung des Rohres 13 und der Stange 15 ist der Marknagel 11 insgesamt nicht geradlinig, sondern gekrümmt ausgebildet. Die Krümmung des Marknagels 11 kann insbesondere einer physiologischen Krümmung eines zu behandelnden Röhrenknochens entsprechen, so dass auch eine zum Einsetzen des Marknagels 11 erforderliche Bohrung in einem Markkanal des Röhrenknochens der physiologischen Krümmung des Knochens folgen kann. Bei einem Einsetzen herkömmlicher geradliniger Marknägel sind demgegenüber entsprechend geradlinige Bohrungen erforderlich, so dass die Krümmung eines Knochens nicht berücksichtigt werden und die Bohrung eine Schwächung der Kortikalis zur Folge haben kann. Durch die gekrümmte Ausbildung des Marknagels 11 kann eine solche Schwächung hingegen vermieden werden.

Insbesondere kann der Marknagel 11 als Distraktionsmarknagel ausgebildet und die Stange 15 schrittweise aus dem Rohr 13 heraus bewegbar sein, um den Abstand der mit dem Marknagel 11 verbundenen Knochenteile sukzessive vergrößern und den Knochen durch die sich in der Knochenlücke kontinuierlich bildende Knochensubstanz letztlich verlängern zu können. Ebenso ist es möglich, einen solchen Marknagel 11 als Kontraktionsmarknagel auszubilden, bei welchem die Stange 15 schrittweise in das Rohr 13 hineinbewegt werden kann.

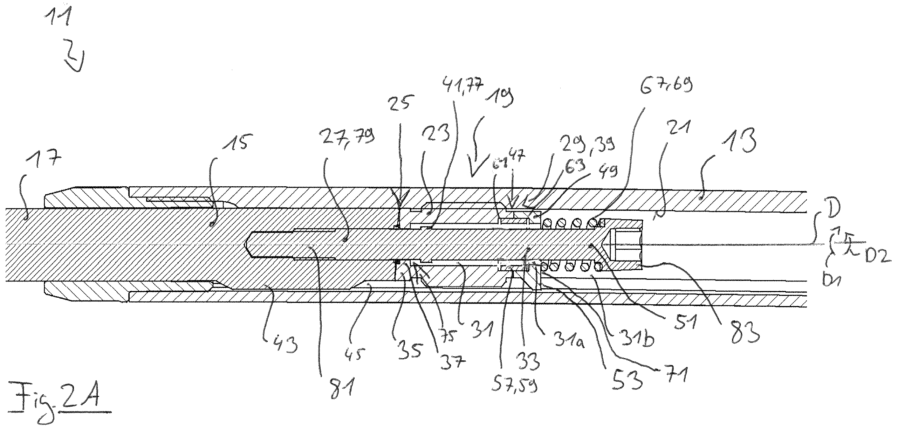

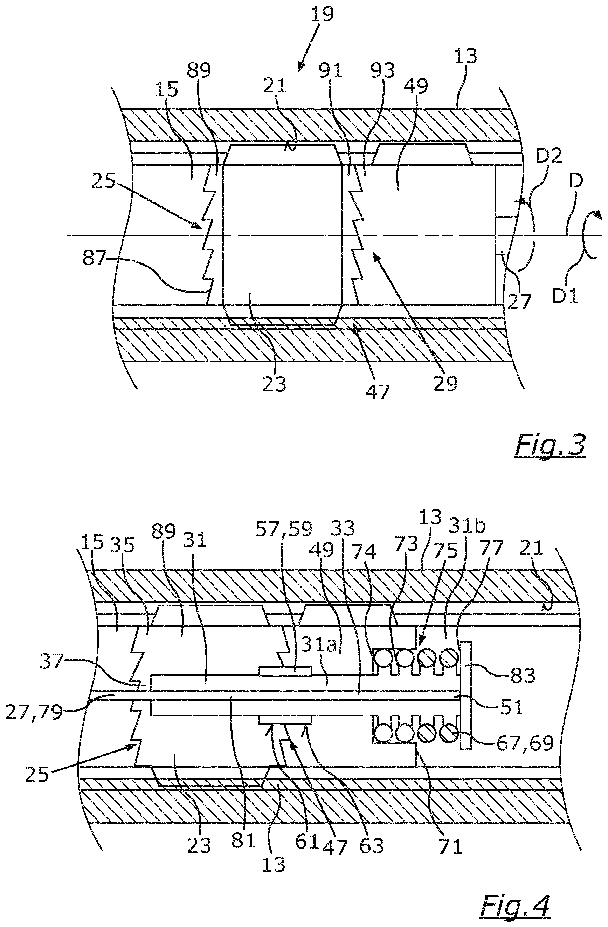

Um eine schrittweise Veränderung der Länge des Marknagels 11 zu ermöglichen, ist innerhalb des Rohres 13 eine Einstellmechanik 19 angeordnet, welche durch wiederholte relative Drehbewegungen zwischen dem Rohr 13 und der Stange 15 betätigbar ist. Wie die Fig. 2A bis 4 zeigen, weist die Einstellmechanik 19 dazu ein in einem Innengewinde 21 des Rohres 13 um eine Drehachse D drehbares Gewindeelement 23 auf, mit welchem die Stange 15 über eine Kopplungsverzahnung 25 in Eingriff steht (vgl. insbesondere Fig. 3). Darüber hinaus umfasst die Einstellmechanik 19 ein Blockierelement 49, welches mit dem Gewindeelement 23 an dessen der Stange 15 abgewandten Seite 29 über eine Blockierverzahnung 47 in Eingriff steht und verdrehsicher innerhalb des Rohres 13 geführt ist. Mit der Stange 15 ist zudem ein Verbindungselement 27 der Einstellmechanik 19 starr verbunden, welches als eine in die Stange 15 eingeschraubte Schraube 79 mit einem Schaft 81 und einem Schraubenkopf 83 ausgebildet ist. Das Verbindungselement 27 erstreckt sich durch das Gewindeelement 23 hindurch und mit einem aus dem Gewindeelement 23 herausragenden Teil 33 in das Blockierelement 49 hinein. Ferner ragt das Verbindungselement 27 mit einem Verlängerungsabschnitt 51 aus dem Blockierelement 49 heraus, so dass der Schraubenkopf 83 von dem Rohr 13 umgeben ist. An einer Unterseite des Schraubenkopfs 83 sowie an einer dem Gewindeelement 23 abgewandten Stirnseite 71 des Blockierelements 49 ist eine als Feder 69 ausgebildete Spanneinrichtung 67 abgestützt, durch welche das Blockierelement 49 in Richtung des Gewindeelements 23 vorgespannt ist.

Wie insbesondere aus Fig. 3 hervorgeht, umfasst die Kopplungsverzahnung 25 eine an der Stange 15 ausgebildete Verzahnung 87 sowie eine an dem Gewindeelement 23 ausgebildete Verzahnung 89, welche in einer Ausgangsstellung der Einstellmechanik 19 in Eingriff zueinanderstehen. Die Verzahnungen 87 und 89 sind derart ausgebildet, dass eine Drehung der Stange 15 relativ zu dem Rohr 13 um die Drehachse D des Gewindeelements 23 entlang einer ersten Drehrichtung D1 auf das Gewindeelement 23 übertragbar ist, so dass das Gewindeelement 23 durch die Drehung der Stange 15 entlang der ersten Drehrichtung D1 gedreht und axial in dem Rohr 13 bewegt werden kann. Durch die Kopplung der Stange 15 mit dem Gewindeelement 23 kann somit auch die Stange 15 durch die Drehung entlang der ersten Drehrichtung D1 relativ zu dem Rohr 13 und insbesondere aus dem Rohr 13 heraus bewegt werden, wobei die durch die Drehung erfolgende Längenänderung durch eine Gewindesteigung des Innengewindes 21 des Rohres 13 bzw. die Gewindesteigung des Gewindeelements 23 sowie den Winkel, um welchen die Stange 15 gedreht wird, vorgegeben sein kann.

Um das Mitnehmen des Gewindeelements 23 bei einer Drehung der Stange 15 entlang der ersten Drehrichtung D1 zu ermöglichen, weist die Blockierverzahnung 47 des verdrehsicher in dem Rohr 13 geführten Blockierelements 49 in der ersten Drehrichtung D1 einen Freilauf auf, so dass das Gewindeelement 23 entlang der ersten Drehrichtung D1 relativ zu dem Blockierelement 49 verdreht werden kann. Dazu umfasst die Blockierverzahnung 47 eine an dem Gewindeelement 23 ausgebildete Verzahnung 91 sowie eine an dem Blockierelement 49 ausgebildete Verzahnung 93, wobei die Steigung der Verzahnungen 91 und 93 in der ersten Drehrichtung D1 so gewählt sind, dass das Blockierelement 49 während einer Drehung des Gewindeelements 23 entlang der ersten Drehrichtung D1 durch die aneinander gleitenden Verzahnungen 91 und 93 axial entgegen der Kraft der Feder 69 zurückgedrängt werden kann (vgl. Fig. 3). Insbesondere kann die Verzahnung 91 infolge einer solchen Drehung um einen Zahn gegenüber der Verzahnung 93 versetzt werden.

Damit die Einstellmechanik 19 kontrolliert betätigt und insbesondere die im Zuge einer Betätigung zu erreichende Längenveränderung des Marknagels 11 exakt vorgeben werden kann, ist eine Drehbewegung der Stange 15 relativ zu dem Rohr 13 um die Drehachse D des Gewindeelements 23 begrenzt. Dazu ist an der Stange 15 eine Passfeder 43 ausgebildet, welche innerhalb des Rohres 13 in einer Nut 45 geführt ist und nach einer Drehung der Stange 15 um einen vorgegebenen Winkel an der Nut 45 anschlägt, so dass auch das Gewindeelement 23 bei einer Drehung der Stange 15 entlang der ersten Drehrichtung D1 um einen exakt vorgegebenen Winkel verdreht wird und der Marknagel 11 eine definierte Längenänderung erfährt. Zudem dient die Nut 45 dazu, das Blockierelement 49 verdrehsicher in dem Rohr 13 zu führen, wozu das Blockierelement 49 ebenfalls eine in der Nut 45 geführte Passfeder 53 aufweist. Die Passfeder 53 ist dabei dicker als die Passfeder 43 ausgebildet, so dass das Blockierelement 49 verdrehsicher, das Gewindeelement 23 hingegen um den vorgegebenen Winkel verdrehbar ist.

Nachdem das Gewindeelement 23 mittels der Stange 15 entlang der ersten Drehrichtung D1 um den durch die Passfeder 43 und die Nut 45 vorgegebenen Winkel verdreht wurde, kann die Stange 15 entlang einer der ersten Drehrichtung D1 entgegengesetzten Drehrichtung D2 zurückgedreht werden, um die Einstellmechanik 19 wieder in die Ausgangsstellung zu versetzen und eine erneute Betätigung der Einstellmechanik 19 zum Verändern der Länge des Marknagels 11 zu ermöglichen. Dazu weist die Kopplungsverzahnung 25 entlang der zweiten Drehrichtung D2 eine Freilaufrichtung auf, während das Blockierelement 49 eine Sperre für das Gewindeelement 23 bezüglich Drehungen entlang der zweiten Drehrichtung D2 bildet. Aufgrund des bezüglich Drehungen entlang der zweiten Drehrichtung D2 durch die Blockierverzahnung 47 blockierten Gewindeelements 23 kann die Stange 15 somit entlang der zweiten Drehrichtung D2 relativ zu dem Gewindeelement 23 gedreht werden, ohne dass das Gewindeelement 23 eine Drehung ausführt oder axial in dem Rohr 13 bewegt wird. Während einer solchen Drehung der Stange 15 entlang der zweiten Drehrichtung D2 gleitet die Verzahnung 87 der Stange 15 an der Verzahnung 89 des Gewindeelements 23, so dass die Stange 15 und das Verbindungselement 27 axial relativ zu dem Gewindeelement 23 und dem Blockierelement 49 bewegt werden und die Feder 69 wiederum komprimiert wird. Insbesondere kann dadurch ein sicheres Schließen der Blockierverzahnung 47 während dieser Drehung der Stange 15 erreicht werden, um das Gewindeelement 23 gegen ein Mitdrehen zu blockieren. Die Sperre der Blockierverzahnung 47 wird zudem durch eine senkrechte Ausrichtung der Verzahnungen 91 und 93 zu der zweiten Drehrichtung D2 erreicht, wobei eine entsprechende Ausrichtung der Verzahnungen 87 und 89 der Kopplungsverzahnung 25 zu der Drehrichtung D1 das Mitnehmen des Gewindeelements 23 bei einer Drehung der Stange 15 entlang der ersten Drehrichtung D1 ermöglicht.

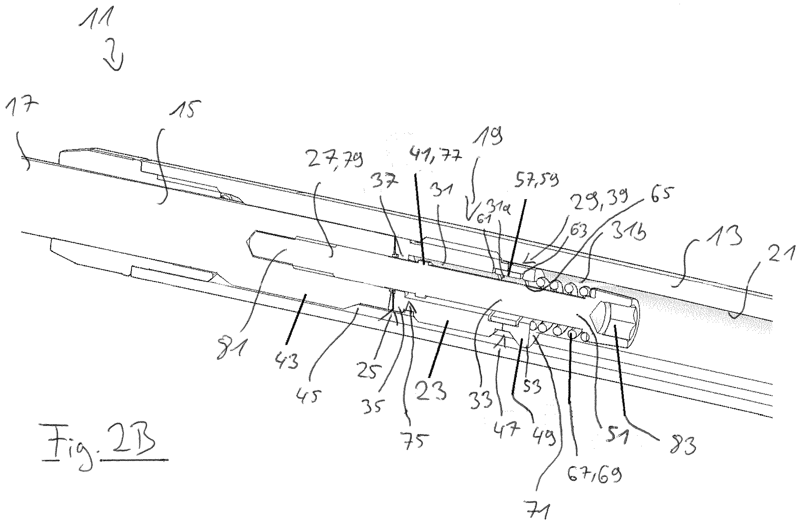

Zusätzlich zu dem Übertragen einer Vorspannung auf das Blockierelement 49 ist das Verbindungselement 27 der Einstellmechanik 19 insbesondere dazu vorgesehen, die Stange 15 mit der Einstellmechanik 19 zu koppeln und dadurch ein ungewolltes Lösen der Stange 15 aus dem Rohr 13 zu verhindern. Wie insbesondere die Fig. 2 und 2B veranschaulichen, ist das Verbindungselement 27 geradlinig ausgebildet und erstreckt sich in der gezeigten Ausgangsstellung der Einstellmechanik 19, in welcher die Kopplungsverzahnung 25 in Eingriff steht, entlang der Drehachse D des Gewindeelements 23. Aufgrund der Krümmung der Stange 15 wird das geradlinig ausgebildete Verbindungselement 27 jedoch bei einer Drehung der Stange 15 um die Drehachse D radial gegenüber der Drehachse D ausgelenkt und führt eine Taumelbewegung aus, welche grundsätzlich eine Belastung des Verbindungselements 27 mit Biegemomenten zur Folge haben könnte, die gegebenenfalls sogar zu einem Brechen des Verbindungselements 27 führen könnten und einer gekrümmten Ausbildung von Marknägeln bisher entgegenstanden.

Um diese Taumelbewegung jedoch zu ermöglichen und eine Belastung des Verbindungselements 27 durch Biegemomente zu verhindern, erstreckt sich das Verbindungselement 27 des hierin offenbarten Marknagels 11 innerhalb des Gewindeelements 23 mit einem radialen Freiraum 31. Dieser radiale Freiraum 31 ist insbesondere derart gewählt, dass das Verbindungselement 27 während einer Drehung der Stange 15 relativ zu dem Rohr 13 um die Drehachse D des Gewindeelements 23 nicht an einer Innenseite des Gewindeelements 23 anschlägt. Auch der aus dem Gewindeelement 23 herausragende Teil 33 des Verbindungselements 27 erstreckt sich in dem Blockierelement 49 mit einem radialen Freiraum 31a, um innerhalb des Blockierelements 49 ebenfalls eine Auslenkung des Verbindungselements 27 gegenüber der Drehachse D während einer Drehung der Stange 15 zu ermöglichen.

Ebenso erstreckt sich der aus dem Blockierelement 49 herausragende Verlängerungsabschnitt 51 des Verbindungselements 27, welcher lediglich von dem Rohr 13 umgeben ist, mit einem radialen Freiraum 31b in dem Rohr 13, so dass auch dieser axial am weitesten von der Stange 15 entfernte Verlängerungsabschnitts 51 des Verbindungselements 27 während einer Drehung der Stange 15 gegenüber der Drehachse D ausgelenkt werden kann, ohne dass der Verlängerungsabschnitt 51 an einer Innenseite oder dem Innengewinde 21 des Rohrs 13 anschlägt und das Verbindungselement 27 eine Biegebelastung erfährt. Insbesondere können der radiale Freiraum 31b und der Schraubenkopf 83 des Verbindungselements 27 insbesondere derart aufeinander abgestimmt sein, dass der Schraubenkopf 83, welcher die größte radiale Auslenkung bezüglich der Drehachse D bei einer Drehung der Stange 15 um die Drehachse D erfährt, bei einer Drehung der Stange 15 nicht mit dem Innengewinde 21 des Rohres 13 in Berührung kommt. Ferner kann der radiale Freiraum 31b größer als der radiale Freiraum 31 und/oder der radiale Freiraum 31a sein, um die mit dem Abstand von der Stange 15 steigende radiale Auslenkung des Verbindungselements 27 zu ermöglichen.

Während das Verbindungselement 27 somit in dem Gewindeelement 23, in dem Blockierelement 49 und in dem Rohr 13 innerhalb der radialen Freiräume 31, 31a und 31b geführt ist, um die erforderliche Taumelbewegung des Verbindungselements 27 zu ermöglichen, ist das Verbindungselement 27 dennoch mittels des Gewindeelements 23 an einem der Stange 15 zugewandten Endabschnitt 35 des Gewindeelements 23 zentriert (vgl. Fig. 2A, 2B und 4). Dazu ist an dem Endabschnitt 35 des Gewindeelements 23 ein radial nach innen ragender Zentrierabschnitt 37 ausgebildet, welcher sich beispielsweise ringartig um das Verbindungselement 27 erstrecken und das Verbindungselement 27 im Wesentlichen radial spielfrei umgeben kann.

Insbesondere ermöglicht es die Zentrierung des Verbindungselements 27 mittels des Zentrierabschnitts 37, auch die Stange, an welcher das Verbindungselement 27 starr befestigt ist, zu dem Gewindeelement 23 zu zentrieren und radial auszurichten. Dadurch kann beispielsweise verhindert werden, dass sich die Stange 15 im Zuge einer Betätigung der Einstellmechanik 19 radial relativ zu dem Gewindeelement 23 bewegt und die Kopplungsverzahnung 25 nicht wieder exakt in Eingriff gelangt. Insbesondere bei einem Zurückdrehen der Stange 15 entlang der zweiten Drehrichtung D2, währenddessen die Stange 15 axial relativ zu dem Gewindeelement 23 bewegt und die Verzahnung 87 um einen Zahn gegenüber der Verzahnung 89 versetzt wird (vgl. Fig. 3), ist die Stange 15 im Moment des Überspringens der Verzahnung 87 nicht durch die Kopplungsverzahnung 25 radial relativ zu dem Gewindeelement 23 ausgerichtet, so dass eine radiale Relativbewegung der Stange 15 zu dem Gewindeelement 23 grundsätzlich möglich wäre. Dies kann jedoch durch die Zentrierung des Verbindungselements 27 mittels des Zentrierabschnitts 37 verhindert werden.

Indem der Zentrierabschnitt 37 dabei jedoch lediglich als ein axial schmaler Abschnitt an dem Endbereich 35 des Gewindeelements 23 ausgebildet ist, erfolgt Zentrierung des Verbindungselements 27 in einem Bereich, in welchem das Verbindungselement 27 lediglich geringfügig gegenüber der Drehachse D des Gewindeelements 23 ausgelenkt wird, wenn die Stange 15 der Drehachse D gedreht wird. Dementsprechend können durch die Zentrierung des Verbindungselements 27 allenfalls kleine und zu tolerierende Biegemomente auf das Verbindungselement 27 übertragen werden, die nicht zu einer hohen Belastung oder gar einem Brechen des Verbindungselements 27 führen können. Zwischen dem Zentrierabschnitt 37, dessen axiale Länge insbesondere etwa 1/7 der axialen Länge des Gewindeelements 23 entspricht, und einer der Stange 15 abgewandten Stirnseite 39 des Gewindeelements 23 erstreckt sich das Verbindungselement 27 hingegen vollständig mit dem radialen Freiraum 31 in dem Gewindeelement 23, so dass die mit zunehmendem Abstand von der Stange 15 größer werdenden Auslenkungen des Verbindungselements 27 während einer Drehung der Stange um die Drehachse D ermöglicht werden.

Ferner ist an dem Schaft 81 des Verbindungselements 27 eine radiale Verbreiterung 77 ausgebildet, welche bei dem anhand der Fig. 2A und 2B veranschaulichten Ausführungsbeispiel als radiale Anschlagsverbreiterung 41 innerhalb des Gewindeelements 23 angeordnet ist. Insbesondere ermöglicht diese radiale Anschlagsverbreiterung 41, eine axiale Relativbewegung zwischen der Stange 15 und dem Rohr 13, bei welcher die Stange 15 aus dem Rohr 13 herausbewegt wird, zu begrenzen, indem die radiale Anschlagsverbreiterung 41 an dem Zentrierabschnitt 37 anschlägt. Dadurch kann die Stange 15 zuverlässig axial in dem Rohr 13 gesichert werden, um beispielsweise ein ungewolltes Lösen der Stange 15 aus dem Rohr 13 während einer Operation zu verhindern. Insofern erfüllt der Zentrierabschnitt 37 eine Doppelfunktion, indem der Zentrierabschnitt 37 einerseits zur Zentrierung des Verbindungselements 27 und der Stange 15 und andererseits zum Sichern der Stange 15 in dem Rohr 13 dient.

Darüber hinaus bilden der Zentrierabschnitt 37 und die radiale Anschlagsverbreiterung 41 gemeinsam einen Anschlag 75, durch welchen auch eine axiale Bewegung der Stange 15 und des daran starr befestigten Verbindungselements 27 entgegen der Kraft der Feder 69 begrenzt wird. Somit dienen die radiale Anschlagsverbreiterung 41 und der Zentrierabschnitt 37 auch dazu, eventuelle Überbelastungen der Feder 69 während eines Einsetzens oder Herausziehens des Marknagels 11 während einer Operation zu verhindern.

Um auch einen sicheren Eingriff der Blockierverzahnung 47 nach einer Drehung des Gewindeelements 23 zu erreichen, ist ferner auch das Blockierelement 49 zu dem Gewindeelement 23 zentriert (vgl. Fig. 2A, 2B und 4). Dazu ist ein Zentrierelement 57 vorgesehen, welches das Blockierelement 47 gegen Bewegungen in radialer Richtung relativ zu dem Gewindeelement 23 blockiert. Dieses Zentrierelement 57 ist insbesondere als eine Zentrierhülse 59 ausgebildet, welche in eine radiale Freistellung 61 an einer Innenseite des Gewindeelements 61 eingesteckt ist. Auch das Blockierelement 49 weist an seiner Innenseite eine radiale Freistellung 63 auf, so dass das Blockierelement 49 auf die Zentrierhülse 59 aufgesteckt werden kann und die in den Freistellungen 61 und 63 angeordnete Zentrierhülse 59 das Gewindeelement 23 radial mit dem Blockierelement 49 verbindet. Aufgrund der Freistellungen 61 und 63 kann die Zentrierhülse 59 derart angeordnet werden, dass der radiale Freiraum 31 in dem Gewindeelement 23 bzw. der radiale Freiraum 31a in dem Blockierelement 49 für das Verbindungselement 27 nicht beschränkt wird. Auch hierbei kann durch die Zentrierhülse 59 insbesondere eine radiale Auslenkung des Blockierelements 49 relativ zu dem Gewindeelement 23 in dem Moment verhindert werden, in dem die Verzahnungen 91 und 93 während einer Drehung des Gewindeelements 23 entlang der ersten Drehrichtung D1 außer Eingriff zueinander geraten (vgl. auch Fig. 3).

Fig. 4 veranschaulicht schematisch eine weitere Ausführungsform des Marknagels 11, welche grundsätzlich wie vorstehend erläutert ausgebildet ist. Jedoch ist die Feder 69, durch welche das Blockierelement 49 in Richtung des Gewindeelements 23 vorgespannt ist, bei dieser Ausführungsform nicht an der Stirnseite 71 des Blockierelements 49 abgestützt, sondern das Blockierelement 49 weist eine Federaufnahme 73 auf, an deren Boden 74 die Feder 69 abgestützt ist. Beispielsweise kann die Federaufnahme 63 als eine Bohrung in dem Blockierelement 49 ausgebildet sein.

Die Ausbildung des Blockierelements 49 mit der Federaufnahme 73 ermöglicht es insbesondere, den Abstand zwischen dem Schraubenkopf 83 und dem Blockierelement 49 in axialer Richtung zu minimieren, so dass auch die Auslenkung des Verbindungselements 27 gegenüber der Drehachse D des Gewindeelements 23 begrenzt werden kann. Zudem kann das Verbindungselement 27, wie Fig. 4 zeigt, ohne die radiale Anschlagsverbreiterung 41 ausgebildet werden und der Anschlag 75 zum Begrenzen der Belastung der Feder 69 kann durch die dem Gewindeelement abgewandte Stirnseite 71 des Blockierelements 49 und den Schraubenkopf 83 gebildet werden, welcher insofern eine radiale Verbreiterung 77 des Verbindungselements 27 darstellt. Ebenso kann das Zusammenwirken der Stirnseite 71 des Blockierelements 49 mit dem Schraubenkopf 83 ein Lösen der Stange 15 aus dem Rohr 13 zuverlässig verhindern.

### Bezugszeichenliste

- 11: Marknagel
- 13: Rohr
- 15: Stange
- 17: Befestigungsabschnitt
- 19: Einstellmechanik
- 21: Innengewinde
- 23: Gewindeelement
- 25: Kopplungsverzahnung
- 27: Verbindungselement
- 29: Seite des Gewindeelements
- 31: radialer Freiraum
- 31a: radialer Freiraum
- 31b: radialer Freiraum
- 33: aus dem Gewindeelement herausragender Teil des Verbindungselements
- 35: Endabschnitt des Gewindeelements
- 37: Zentrierabschnitt
- 39: der Stange abgewandte Stirnseite des Gewindeelements
- 41: radiale Anschlagsverbreiterung
- 43: Passfeder der Stange
- 45: Nut
- 47: Blockierverzahnung
- 49: Blockierelement
- 51: Verlängerungsabschnitt des Verbindungselements
- 53: Passfeder des Blockierelements
- 57: Zentrierelement
- 59: Zentrierhülse
- 61: radiale Freistellung
- 63: radiale Freistellung
- 67: Spanneinrichtung
- 69: Feder
- 71: Stirnseite des Blockierelements
- 73: Federaufnahme
- 74: Boden
- 75: Anschlag
- 77: radiale Verbreiterung des Verbindungselements
- 79: Schraube
- 81: Schaft
- 83: Schraubenkopf
- 85: Befestigungsöffnung
- 87: Verzahnung
- 89: Verzahnung
- 91: Verzahnung
- 93: Verzahnung
- D: Drehachse
- D1: erste Drehrichtung
- D2: zweite Drehrichtung

## Patentansprüche

1. Gekrümmter Marknagel (11), insbesondere Distraktionsmarknagel oder Kontraktionsmarknagel, zum Verbinden zweier Teile eines Knochens, insbesondere zum sukzessiven Verlängern oder zum sukzessiven Verkürzen eines Knochens,
mit einem gekrümmten Rohr (13) zum Befestigen an einem ersten Knochenteil und
mit einer innerhalb des Rohres (13) angeordneten gekrümmten Stange (15), welche einen aus dem Rohr (13) herausragenden Befestigungsabschnitt (17) zum Befestigen an einem zweiten Knochenteil aufweist, wobei innerhalb des Rohres (13) eine Einstellmechanik (19) angeordnet ist, welche durch wiederholte relative Drehbewegungen zwischen dem Rohr (13) und der Stange (15) betätigbar ist,
wobei die Stange (15) durch Betätigen der Einstellmechanik (19) aus dem Rohr (13) herausfahrbar oder in das Rohr (13) hineinfahrbar ist,
wobei die Einstellmechanik (19) ein in einem Innengewinde (21) des Rohres (13) um eine Drehachse (D) drehbares Gewindeelement (23) umfasst, mit welchem die Stange (15) über eine Kopplungsverzahnung (25) in Eingriff steht, und
wobei die Einstellmechanik (19) ein starr an der Stange (15) befestigtes Verbindungselement (27) aufweist,
wobei sich das Verbindungselement (27) durch das Gewindeelement (23) hindurch erstreckt und an einer der Stange (15) abgewandten Seite (29) aus dem Gewindeelement (23) herausragt, und
wobei sich das Verbindungselement (27) innerhalb des Gewindeelements (23) mit einem radialen Freiraum (31) erstreckt.

2. Gekrümmter Marknagel (11) nach Anspruch 1,
wobei sich ein aus dem Gewindeelement (23) herausragender Teil (33) des Verbindungselements (27) mit einem radialen Freiraum (31a, 31b) in dem Rohr (13) erstreckt; und/oder
wobei das Verbindungselement (27) geradlinig ausgebildet ist.

3. Gekrümmter Marknagel (11) nach Anspruch 1 oder 2,
wobei das Verbindungselement (27) an einem der Stange (15) zugewandten Endabschnitt (35) des Gewindeelements (23) mittels des Gewindeelements (23) zentriert ist,
insbesondere wobei das Gewindeelement (23) zum Zentrieren des Verbindungselements (27) einen radial nach innen ragenden Zentrierabschnitt (37) aufweist, dessen axiale Länge in einem Bereich von 1/10 bis 1/3, insbesondere 1/8 bis 1/4, der axialen Länge des Gewindeelements (23) liegt oder 1/7 der axialen Länge des Gewindeelements (23) beträgt.

4. Gekrümmter Marknagel (11) nach Anspruch 3,
wobei sich das Verbindungselement (27) zwischen dem der Stange (15) zugewandten Endabschnitt (35) des Gewindeelements (23) und einer der Stange (15) abgewandten Stirnseite (39) des Gewindeelements (23) vollständig mit einem radialen Freiraum (31) in dem Gewindeelement (23) erstreckt; und/oder
wobei das Verbindungselement (27) eine radiale Anschlagsverbreiterung (41) aufweist, wobei der Endabschnitt (35) des Gewindeelements (23) und die Anschlagsverbreiterung (41) des Verbindungselements (27) eine axiale Relativbewegung zwischen der Stange (15) und dem Gewindeelement (23) begrenzen.

5. Gekrümmter Marknagel (11) nach einem der vorhergehenden Ansprüche, wobei eine Drehbewegung der Stange (15) um die Drehachse (D) relativ zu dem Rohr (13) begrenzt ist,
wobei die Stange (15) insbesondere eine Passfeder (43) aufweist, welche innerhalb des Rohres (13) in einer Nut (45) geführt ist, wobei die Nut (45) eine Drehbewegung der Stange (15) um die Drehachse begrenzt.

6. Gekrümmter Marknagel (11) nach einem der vorhergehenden Ansprüche, wobei eine Drehung der Stange (15) um die Drehachse (D) entlang einer ersten Drehrichtung (D1) über die Kopplungsverzahnung (25) auf das Gewindeelement (23) übertragbar ist.

7. Gekrümmter Marknagel (11) nach Anspruch 6,
wobei die Stange (15) in einer der ersten Drehrichtung (D1) entgegengesetzten zweiten Drehrichtung (D2) relativ zu dem Gewindeelement (23) um die Drehachse (D) drehbar ist.

8. Gekrümmter Marknagel (11) nach Anspruch 7,
wobei das Gewindeelement (23) an einer der Stange (15) abgewandten Seite über eine Blockierverzahnung (47) mit einem Blockierelement (49) in Eingriff steht,
wobei das Verbindungselement (27) durch das Blockierelement (49) hindurch geführt ist und mit einem Verlängerungsabschnitt (51) an einer dem Gewindeelement (23) abgewandten Seite aus dem Blockierelement (49) herausragt, und
wobei sich das Verbindungselement (27) mit einem radialen Freiraum (31a) in dem Blockierelement (49) erstreckt.

9. Gekrümmter Marknagel (11) nach Anspruch 8,
wobei das Blockierelement (49) verdrehsicher in dem Rohr (13) geführt ist, insbesondere wobei das Blockierelement (49) eine Passfeder (53) aufweist, welche innerhalb des Rohres (13) in einer Nut (45) geführt ist; und/oder
wobei die Blockierverzahnung (47) in der ersten Drehrichtung (D1) einen Freilauf aufweist, durch welchen das Gewindeelement (23) für eine Drehung entlang der ersten Drehrichtung (D1) relativ zu dem Blockierelement (49) freigegeben ist, und wobei die Blockierverzahnung (47) in der zweiten Drehrichtung (D2) eine Sperre bildet, welche eine Drehung des Gewindeelements (23) relativ zu dem Blockierelement (49) entlang der zweiten Drehrichtung (D2) blockiert,
wobei die Kopplungsverzahnung (25) in der zweiten Drehrichtung (D2) insbesondere einen Freilauf aufweist, welcher eine Drehung der Stange (15) relativ zu dem durch die Blockierverzahnung (47) blockierten Gewindeelement (23) entlang der zweiten Drehrichtung (D2) ermöglicht.

10. Gekrümmter Marknagel (11) nach Anspruch 8 oder 9,
wobei die Einstellmechanik (19) ein Zentrierelement (57) umfasst, welches das Blockierelement (49) gegen Bewegungen in radialer Richtung relativ zu dem Gewindeelement (23) blockiert,
insbesondere wobei das Zentrierelement (57) als eine Zentrierhülse (59) ausgebildet ist, auf oder in die das Blockierelement (49) und das Gewindeelement (23) steckbar sind; und/oder
insbesondere wobei das Blockierelement (49) und das Gewindeelement (23) an ihrer Innenseite oder ihrer Außenseite eine jeweilige radiale Freistellung (61, 63) aufweisen, wobei das Zentrierelement (57) in den radialen Freistellungen (61, 63) angeordnet ist und eine axiale Verbindung zwischen dem Gewindeelement (23) und dem Blockierelement (49) bildet.

11. Gekrümmter Marknagel (11) nach einem der Ansprüche 8 bis 10,
wobei das Blockierelement (49) über das Verbindungselement (27) in Richtung des Gewindeelements (23) vorgespannt ist,
insbesondere wobei das Blockierelement (49) entgegen der Rückstellkraft der Vorspannung außer Eingriff mit dem Gewindeelement (23) bringbar ist.

12. Gekrümmter Marknagel (11) nach Anspruch 11,
wobei mit dem Verbindungselement (27) eine Spanneinrichtung (67), insbesondere eine Feder (69), gekoppelt ist, welche das Blockierelement (49) in Richtung des Gewindeelements (23) vorspannt.

13. Gekrümmter Marknagel (11) nach Anspruch 12,
wobei die Spanneinrichtung (67) eine Feder (69) umfasst oder als eine Feder (69) ausgebildet ist, welche an dem Blockierelement (49) und an dem Verlängerungsabschnitt (51) des Verbindungselements (27) abgestützt ist, insbesondere wobei die Feder (69) an einer dem Gewindeelement (23) abgewandten Stirnseite (71) des Blockierelements (49) abgestützt ist; oder insbesondere wobei das Blockierelement (49) eine Federaufnahme (73), insbesondere eine Bohrung oder einen Schlitz, aufweist, in welcher die Feder (69) zumindest teilweise aufgenommen ist, wobei die Feder (69) in Richtung des Gewindeelements (23) an einem Boden (74) der Federaufnahme (73) abgestützt ist.

14. Gekrümmter Marknagel (11) nach Anspruch 12 oder 13,
wobei die Einstellmechanik (19) einen Anschlag (75) aufweist, welcher eine axiale Bewegung des Verbindungselements (27) entgegen der Kraft der Spanneinrichtung (67) begrenzt.

15. Gekrümmter Marknagel (11) nach Anspruch 14,
wobei der Anschlag (75) eine radiale Verbreitung (77) des Verbindungselements (27) umfasst;
insbesondere wobei die radiale Verbreiterung (77) des Verbindungselements (27) von einer Anschlagsverbreiterung (41) an einem sich innerhalb des Gewindeelements (23) erstreckenden Abschnitt des Verbindungselements (27) gebildet ist, wobei die Anschlagsverbreiterung (41) und ein radial nach innen gerichteter Zentrierabschnitt (37) des Gewindeelements (23) den Anschlag (75) der Einstellmechanik (19) bilden; oder
insbesondere wobei die radiale Verbreiterung (77) des Verbindungselements (27) an dem Verlängerungsabschnitt (51) des Verbindungselements (27) ausgebildet ist, wobei die radiale Verbreiterung (77) und eine dem Gewindeelement (23) abgewandte Stirnseite (71) des Blockierelements (49) den Anschlag (75) der Einstellmechanik (19) bilden.

## Claims

1. A curved intramedullary nail (11), in particular a distraction intramedullary nail or a contraction intramedullary nail, for connecting two parts of a bone, in particular for successively extending or for successively shortening a bone, said curved intramedullary nail (11) comprising
a curved tube (13) for fastening to a first bone part; and
a curved rod (15) which is arranged within the tube (13) and which has a fastening section (17) projecting from the tube (13) for fastening to a second bone part,
wherein a setting mechanism (19) is arranged within the tube (18) and can be actuated by repeated relative rotational movements between the tube (13) and the rod (15),
wherein the rod (15) can be moved out of the tube (13) or can be moved into the tube (13) by actuating the setting mechanism (19),
wherein the setting mechanism (19) comprises a threaded element (23) which is rotatable about an axis of rotation (D) in an internal thread (21) of the tube (13) and with which the rod (15) is in engagement via a coupling toothed arrangement (25), and
wherein the setting mechanism (19) has a connection element (27) rigidly fastened to the rod (15),
wherein the connection element (27) extends through the threaded element (23) and projects from the threaded element (23) at a side (29) facing away from the rod (15), and
wherein the connection element (27) extends within the threaded element (23) with a radial free space (31).

2. A curved intramedullary nail (11) according to claim 1,
wherein a part (33) of the connection element (27) that projects from the threaded element (23) extends with a radial free space (31a, 31b) in the tube (13); and/or
wherein the connection element (27) is formed in a straight line.

3. A curved intramedullary nail (11) according to claim 1 or 2,
wherein the connection element (27) is centered at an end section (35) of the threaded element (23), said end section facing the rod (15), by means of the threaded element (23),
in particular wherein the threaded element (23) has a radially inwardly projecting centering section (37) for centering the connection element (27), the axial length of said centering section (37) being in a range from 1/10 to 1/3, in particular 1/8 to 1/4, of the axial length of the threaded element (23) or amounting to 1/7 of the axial length of the threaded element (23).

4. A curved intramedullary nail (11) according to claim 3,
wherein the connection element (27) extends completely with a radial free space (31) in the threaded element (23) between the end section (35) of the threaded element (23) that faces the rod (15) and an end face (39) of the threaded element (23) that faces away from the rod (15); and/or
wherein the connection element (27) has a radial abutment widening (41), wherein the end section (35) of the threaded element (23) and the abutment widening (41) of the connection element (27) limit an axial relative movement between the rod (15) and the threaded element (23).

5. A curved intramedullary nail (11) according to any one of the preceding claims,
wherein a rotational movement of the rod (15) about the axis of rotation (D) relative to the tube (13) is limited,
wherein the rod (15) in particular has a key (43) which is guided within the tube (13) in a groove (45), wherein the groove (45) limits a rotational movement of the rod (15) about the axis of rotation.

6. A curved intramedullary nail (11) according to any one of the preceding claims,
wherein a rotation of the rod (15) about the axis of rotation (D) along a first direction of rotation (D1) can be transmitted to the threaded element (23) via the coupling toothed arrangement (25).

7. A curved intramedullary nail (11) according to claim 6,
wherein the rod (15) is rotatable relative to the threaded element (23) about the axis of rotation (D) in a second direction of rotation (D2) opposite the first direction of rotation (D1).

8. A curved intramedullary nail (11) according to claim 7,
wherein the threaded element (23) is in engagement with a blocking element (49) at a side facing away from the rod (15) via a blocking toothed arrangement (47),
wherein the connection element (27) is guided through the blocking element (49) and projects out of the blocking element (49) with an extension section (51) at a side facing away from the threaded element (23), and
wherein the connection element (27) extends with a radial free space (31a) in the blocking element (49).

9. A curved intramedullary nail (11) according to claim 8,
wherein the blocking element (49) is guided in a manner secure against rotation in the tube (13), in particular wherein the blocking element (49) has a key (53) which is guided within the tube (13) in a groove (45); and/or wherein the blocking toothed arrangement (47) has a freewheel in the first direction of rotation (D1), by which freewheel the threaded element (23) is released for a rotation along the first direction of rotation (D1) relative to the blocking element (49), and wherein the blocking toothed arrangement (47) forms a detent in the second direction of rotation (D2) that blocks a rotation of the threaded element (23) relative to the blocking element (49) along the second direction of rotation (D2),
wherein the coupling toothed arrangement (25) in particular has a freewheel in the second direction of rotation (D2) that enables a rotation of the rod (15) relative to the threaded element (23), which is blocked by the blocking toothed arrangement (47), along the second direction of rotation (D2).

10. A curved intramedullary nail (11) according to claim 8 or 9,
wherein the setting mechanism (19) comprises a centering element (57) which blocks the blocking element (49) against movements in a radial direction relative to the threaded element (23),
in particular wherein the centering element (57) is configured as a centering sleeve (59) onto or into which the blocking element (49) and the threaded element (23) can be plugged; and/or
in particular wherein the blocking element (49) and the threaded element (23) have a respective radial opening (61, 63) at their inner side or their outer side, wherein the centering element (57) is arranged in the radial openings (61, 63) and forms an axial connection between the threaded element (23) and the blocking element (49).

11. A curved intramedullary nail (11) according to any one of the claims 8 to 10, wherein the blocking element (49) is preloaded towards the threaded element (G) via the connection element (27),
in particular wherein the blocking element (49) can be brought out of engagement with the threaded element (23) against the return force of the preload.

12. A curved intramedullary nail (11) according to claim 11,
wherein a tensioning device (67), in particular a spring (69), is coupled to the connection element (27) and preloads the blocking element (49) towards the threaded element (23).

13. A curved intramedullary nail (11) according to claim 12,
wherein the tensioning device (67) comprises a spring (69) or is configured as a spring (69) which is supported at the blocking element (49) and at the extension section (51) of the connection element (27),
in particular wherein the spring (69) is supported at an end face (71) of the blocking element (49) that faces away from the threaded element (23); or in particular wherein the blocking element (49) has a spring receiver (73), in particular a bore or a slot, in which the spring (69) is at least partly received, wherein the spring (69) is supported in the direction of the threaded element (23) at a base (74) of the spring receiver (73).

14. A curved intramedullary nail (11) according to claim 12 or 13,
wherein the setting mechanism (19) has an abutment (75) which limits an axial movement of the connection element (27) against the force of the tensioning device (67).

15. A curved intramedullary nail (11) according to claim 14,
wherein the abutment (75) comprises a radial widening (77) of the connection element (27),
in particular wherein the radial widening (77) of the connection element (27) is formed by an abutment widening (41) at a section of the connection element (27) that extends within the threaded element (23), wherein the abutment widening (41) and a radially inwardly directed centering section (37) of the threaded element (23) form the abutment (75) of the setting mechanism (19); or
in particular wherein the radial widening (77) of the connection element (27) is formed at the extension section (51) of the connection element (27), wherein the radial widening (77) and an end face (71) of the blocking element (49) that faces away from the threaded element (23) form the abutment (75) of the setting mechanism (19).

## Revendications

1. Clou intramédullaire incurvé (11), en particulier clou intramédullaire de distraction ou clou intramédullaire de contraction, destiné à relier deux parties d'un os, en particulier destiné à allonger successivement ou à raccourcir successivement un os,
comprenant un tube incurvé (13) destiné à être fixé à une première partie de l'os, et
comprenant une tige incurvée (15) disposée à l'intérieur du tube (13), laquelle présente une portion de fixation (17) dépassant du tube (13) et destinée à être fixée à une deuxième partie de l'os,
dans lequel
un mécanisme de réglage (19) est disposé à l'intérieur du tube (13), qui peut être actionné par des mouvements de rotation relatifs répétés entre le tube (13) et la tige (15),
la tige (15) peut être extraite du tube (13) ou introduite dans le tube (13) par actionnement du mécanisme de réglage (19),
le mécanisme de réglage (19) comprend un élément fileté (23) qui peut tourner autour d'un axe de rotation (D) dans un taraudage (21) du tube (13) et avec lequel la tige (15) est en prise par l'intermédiaire d'une denture de couplage (25), et
le mécanisme de réglage (19) comprend un élément de liaison (27) fixé de manière rigide à la tige (15),
l'élément de liaison (27) s'étend à travers l'élément fileté (23) et dépasse de l'élément fileté (23) sur un côté (29) détourné de la tige (15), et
l'élément de liaison (27) s'étend avec un jeu radial (31) à l'intérieur de l'élément fileté (23).

2. Clou intramédullaire incurvé (11) selon la revendication 1,
dans lequel une partie (33) de l'élément de liaison (27) dépassant de l'élément fileté (23) s'étend avec un jeu radial (31a, 31b) dans le tube (13) ; et/ou
l'élément de liaison (27) est rectiligne.

3. Clou intramédullaire incurvé (11) selon la revendication 1 ou 2,
dans lequel l'élément de liaison (27) est centré au moyen de l'élément fileté (23) au niveau d'une portion d'extrémité (35) de l'élément fileté (23) tournée vers à la tige (15),
en particulier, pour centrer l'élément de liaison (27), l'élément fileté (23) présente une portion de centrage (37) qui s'étend radialement vers l'intérieur et dont la longueur axiale est comprise dans une plage de 1/10 à 1/3, en particulier de 1/8 à 1/4, de la longueur axiale de l'élément fileté (23), ou est égale à 1/7 de la longueur axiale de l'élément fileté (23).

4. Clou intramédullaire incurvé (11) selon la revendication 3,
dans lequel, entre la portion d'extrémité (35) de l'élément fileté (23) tournée vers la tige (15) et une face frontale (39) de l'élément fileté (23) détournée de la tige (15), l'élément de liaison (27) s'étend entièrement avec un jeu radial (31) dans l'élément fileté (23) ; et/ou
l'élément de liaison (27) présente un élargissement de butée radial (41), la portion d'extrémité (35) de l'élément fileté (23) et l'élargissement de butée (41) de l'élément de liaison (27) limitant le mouvement axial relatif entre la tige (15) et l'élément fileté (23).

5. Clou intramédullaire incurvé (11) selon l'une des revendications précédentes,
dans lequel le mouvement de rotation de la tige (15) autour de l'axe de rotation (D) par rapport au tube (13) est limité,
en particulier, la tige (15) comporte une clavette (43) guidée dans une rainure (45) à l'intérieur du tube (13), la rainure (45) limitant le mouvement de rotation de la tige (15) autour de l'axe de rotation.

6. Clou intramédullaire incurvé (11) selon l'une des revendications précédentes,
dans lequel la rotation de la tige (15) autour de l'axe de rotation (D) selon un premier sens de rotation (D1) peut être transmise à l'élément fileté (23) par l'intermédiaire de la denture de couplage (25).

7. Clou intramédullaire incurvé (11) selon la revendication 6,
dans lequel la tige (15) peut tourner autour de l'axe de rotation (D) par rapport à l'élément fileté (23) dans un deuxième sens de rotation (D2) opposé au premier sens de rotation (D1).

8. Clou intramédullaire incurvé (11) selon la revendication 7,
dans lequel l'élément fileté (23) est en prise avec un élément de blocage (49) par l'intermédiaire d'une denture de blocage (47), sur un côté détourné de la tige (15),
l'élément de liaison (27) est mené à travers l'élément de blocage (49) et dépasse de l'élément de blocage (49) par une portion de rallonge (51) sur un côté détourné de l'élément fileté (23), et
l'élément de liaison (27) s'étend avec un jeu radial (31a) dans l'élément de blocage (49).

9. Clou intramédullaire incurvé (11) selon la revendication 8,
dans lequel l'élément de blocage (49) est guidé dans le tube (13) de manière à ne pas pouvoir tourner,
en particulier, l'élément de blocage (49) comporte une clavette (53) qui est guidée dans une rainure (45) à l'intérieur du tube (13) ; et/ou
la denture de blocage (47) présente une roue libre dans le premier sens de rotation (D1), par laquelle l'élément fileté (23) est libéré pour tourner par rapport à l'élément de blocage (49) selon le premier sens de rotation (D1), et la denture de blocage (47) forme un blocage dans le deuxième sens de rotation (D2), qui empêche l'élément fileté (23) de tourner par rapport à l'élément de blocage (49) selon le deuxième sens de rotation (D2),
en particulier, la denture de couplage (25) présente une roue libre dans le deuxième sens de rotation (D2), qui permet à la tige (15) de tourner par rapport à l'élément fileté (23), bloqué par la denture de blocage (47), selon le deuxième sens de rotation (D2).

10. Clou intramédullaire incurvé (11) selon la revendication 8 ou 9,
dans lequel le mécanisme de réglage (19) comprend un élément de centrage (57) qui empêche l'élément de blocage (49) de se déplacer en direction radiale par rapport à l'élément fileté (23),
en particulier, l'élément de centrage (57) est conçu comme un manchon de centrage (59) sur ou dans lequel l'élément de blocage (49) et l'élément fileté (23) peuvent être enfichés ; et/ou
en particulier, l'élément de blocage (49) et l'élément fileté (23) présentent chacun un évidement radial (61, 63) sur leur face intérieure ou sur leur face extérieure, l'élément de centrage (57) étant disposé dans les évidements radiaux (61, 63) et formant une liaison axiale entre l'élément fileté (23) et l'élément de blocage (49).

11. Clou intramédullaire incurvé (11) selon l'une des revendications 8 à 10, dans lequel l'élément de blocage (49) est précontraint vers l'élément fileté (23) par l'intermédiaire de l'élément de liaison (27),
en particulier, l'élément de blocage (49) peut être désengagé de l'élément fileté (23) à l'encontre de la force de rappel exercée par la précontrainte.

12. Clou intramédullaire incurvé (11) selon la revendication 11,
dans lequel un dispositif de serrage (67), en particulier un ressort (69), est couplé à l'élément de liaison (27), lequel met sous précontrainte l'élément de blocage (49) vers l'élément fileté (23).

13. Clou intramédullaire incurvé (11) selon la revendication 12,
dans lequel le dispositif de serrage (67) comprend un ressort (69) ou est conçu comme un ressort (69) supporté par l'élément de blocage (49) et par la partie de rallonge (51) de l'élément de liaison (27),
en particulier, le ressort (69) est supporté par une face frontale (71) de l'élément de blocage (49) détournée de l'élément fileté (23) ; ou
en particulier, l'élément de blocage (49) présente un logement de ressort (73), en particulier un alésage ou une fente, dans lequel le ressort (69) est logé au moins en partie, le ressort (69) étant supporté, en direction de l'élément fileté (23), par une base (74) du logement de ressort (73).

14. Clou intramédullaire incurvé (11) selon la revendication 12 ou 13,
dans lequel le mécanisme de réglage (19) comporte une butée (75) qui limite le mouvement axial de l'élément de liaison (27) à l'encontre de la force exercée par le dispositif de serrage (67).

15. Clou intramédullaire incurvé (11) selon la revendication 14,
dans lequel la butée (75) comprend un élargissement radial (77) de l'élément de liaison (27) ;
en particulier, l'élargissement radial (77) de l'élément de liaison (27) est formé par un élargissement de butée (41) situé sur une portion de l'élément de liaison (27) qui s'étend à l'intérieur de l'élément fileté (23), et l'élargissement de butée (41) et une portion de centrage (37) de l'élément fileté (23) dirigée radialement vers l'intérieur forment la butée (75) du mécanisme de réglage (19) ; ou
en particulier, l'élargissement radial (77) de l'élément de liaison (27) est formé sur la portion de rallonge (51) de l'élément de liaison (27), et l'élargissement radial (77) et une face frontale (71) de l'élément de blocage (49) détournée de l'élément fileté (23) forment la butée (75) du mécanisme de réglage (19).
